(19) **Europäisches Patentamt / European Patent Office / Office européen des brevets**

(11) **EP 4 696 144 A2**

(12) # EUROPEAN PATENT APPLICATION

(43) Date of publication:
18.02.2026 Bulletin 2026/08

(21) Application number: 25218820.6

(22) Date of filing: 10.03.2021

(51) International Patent Classification (IPC):
**A23J 3/34** (2006.01)

(52) Cooperative Patent Classification (CPC):
**A23J 3/16; A23J 1/125; A23J 1/148; A23J 3/14;
A23J 3/18; A23J 3/346; A23K 10/12; A23K 10/18;
A23K 20/147; A23K 20/163; A23K 50/80;
A23L 11/50; C12N 1/14; C12N 1/145; C12N 1/16;**
(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 07.06.2020 US 202063035797 P
08.06.2020 US 202063036274 P
16.06.2020 US 202063039694 P
16.07.2020 US 202063052745 P
09.11.2020 US 202017093557

(62) Document number(s) of the earlier application(s) in
accordance with Art. 76 EPC:
**21823074.6 / 4 161 284**

(71) Applicant: **Prairie Aquatech LLC
Brookings, SD 57006 (US)**

(72) Inventors:
• **Harstad Dennis Brookings, SD 57006 / US,
Dennis**
**Brookings, 57006 (US)**
• **Nates, Sergio F.**
**Haymarket, 20169 (US)**

(74) Representative: **advotec.
Patent- und Rechtsanwaltspartnerschaft mbB
Bahnhofstraße 5
94315 Straubing (DE)**

Remarks:
•The complete document including Reference
Table(s) and the Sequence Listing(s) can be
downloaded from the EPO website
•This application was filed on 26-11-2025 as a
divisional application to the application mentioned
under INID code 62.

(54) **MICROBIAL-BASED PROCESS FOR IMPROVED QUALITY PROTEIN CONCENTRATE**

(57) The invention relates to a method of treating plant-based material comprising:

a) transferring said plant-based material to a first mix-tank (102(a)), wherein said plant-based material is mixed with one or more first solvents to produce a washed mash;

b) separating the washed mash into at least one centrate and a washed cake;

c) transferring said washed cake to one or more second mix-tanks (102(b)-102(d)), wherein one or more second solvents are mixed with said washed cake to produce a washed cake suspension;

d) transferring said washed cake suspension to one or more fermenters (104), wherein said transferred washed cake suspension is inoculated with at least one microbe, and wherein said inoculated washed cake suspension is incubated for a sufficient time to produce a fermented mixture;

e) optionally heating said fermented mixture for a time sufficient to achieve a degree of hydrolysis (DH) of at least 10% of the proteins therein;

f) separating said fermented mixture into a fermented centrate and a fermented cake by hydrodynamic force;

g) transferring the fermented centrate to:

(i) a first mix-tank (102(a)) and/or

(ii) one or more second mix-tanks (102(b)-102(d)),

wherein a mix-tank comprises said plant-based material or said washed cake, and wherein steps (c)-(f) and h) are repeated at least one (1) time for sub-steps (i) or (ii); and

h) drying said fermented cake,

wherein the resulting dried fermented cake has higher protein content compared to said transferred plant-based material.

EP 4 696 144 A2

**FIG. 1**

(52) Cooperative Patent Classification (CPC): (Cont.)
**C12N 1/165;** A23L 11/45; C12R 2001/645;
Y02A 40/818; Y02P 60/87

**Description**

## BACKGROUND OF THE INVENTION

FIELD OF THE INVENTION

The invention generally relates to incubation processes, and specifically microbial-based incubation processes to produce high quality protein concentrates, including products made therefrom and use of such products in the formulation of nutrient feeds.

BACKGROUND INFORMATION

In 2008, approximately 28% of the world's wild, marine fish stocks were overexploited and 52% were fully exploited, even as the demand for per capita consumption of fish and shellfish products have increased with the increasing human population. With dwindling wild fish stocks, in an effort to meet this increased demand, commercial aquaculture production has increased dramatically. However, one of the primary constituents of dietary formulations for aquaculture, fish meal protein, is also derived from wild capture fisheries. It was estimated that at least 6.7 mmt of fish meal will be required to support commercial aquaculture production by 2012. This is clearly unsustainable based on present trends.

Lower cost, more sustainable plant-derived sources of protein have been used to partially replace fish meal in aquaculture diets. Defatted soybean meal (SBM, 42-48% protein) has commonly been used to replace up to 20% of total protein in grower diets for several species, while soy protein concentrate (SPC, 65% protein) has been tested successfully at higher total protein replacement levels, largely governed by the trophic status of the species. These soybean products provide high protein and relatively good amino acid profiles, but are still deficient in some critical amino acids (e.g., taurine) required by carnivorous marine fishes. SPC can be used at higher levels than soybean meal, primarily because the solvent extraction process used to produce SPC removes anti-nutritional factors (e.g., oligosaccharides) and thereby increases protein bioavailability. In addition, a thermal step has been used to inactivate heat-labile antigenic factors. The primary limitations of the current solvent extraction process are its cost, the lack of use for the oligosaccharides removed in the process, and quality issues that frequently limit inclusion to 50% of total protein in the diet. Further, processing of soy material into soybean meal or soy protein concentrates can be environmentally problematic (e.g., problems with disposal of chemical waste associated with hexane-extraction).

Animal protein producers face a range of sustainability risks. By diversifying into sustainable proteins, these businesses can hedge these risks and enter a fast-growing market.

At the same time, innovation in food technology is accelerating and is creating protein production opportunities with the potential to disrupt the incumbent industry. For intensive animal protein producers, a failure to engage with this innovation is a risk. Diversification into producing alternative (i.e., non-animal) proteins is therefore key for both managing the risks of resource-constrained supply chains and for seizing opportunities for market growth.

Meat alternatives and broader protein alternatives that can act as substitutes for traditional animal-based food are attracting considerable financial investment.

Therefore, a plant-derived protein source which is cost-effective and sustainable, and that is of a high-enough quality to fully or substantially replace more of the animal protein in an animal diet is needed, including the use of such plant-derived source in meat alternatives in general (e.g., for human consumption).

## SUMMARY OF THE INVENTION

The present disclosure relates to an organic, microbially-based system to convert plant material into a highly digestible, concentrated protein source that also contains a microbial gum (exopolysaccharide) binder, including such a concentrated source which is suitable for use as a feed for animals and a foodstuff for human consumption.

In embodiments, a composition is disclosed including a non-animal based protein concentrate, wherein the concentrate contains a fermented plant product containing low-pullulan yielding *A. pullulans* and from at least about 65% to about 75% protein content (dry matter basis), where the protein concentrate exhibits one or more of the properties including a degree of hydrolysis (DH) of at least about 2%, an ash content of up to about 4%, or a potassium and magnesium content of less than about 0.1 ppm.

In one aspect, the *A. pullulans* produces less than about 3.0g/L pullulan when grown in a medium comprising between 0.35 and 0.5 g/L yeast extract.

In another aspect, the non-animal based protein concentrate is isolated from plant material including soybeans, sorghum, peanuts, pulses, Rapeseeds, oats, barley, rye, lupins, fava beans, canola, peas, sesame seeds, cottonseeds, palm kernels, barley, grape seeds, olives, safflowers, sunflowers, copra, corn, coconuts, linseed, hazelnuts, wheat, rice, potatoes, cassavas, legumes, camelina seeds, pennycress seeds, mustard seeds, wheat germ meal, corn gluten meal, corn gluten feed, distillery/brewery by-products, and combinations thereof.

In a related aspect, the plant material is from soybeans in the form of soy flakes or soy meal.

In one aspect, a feed or foodstuff comprising the above composition is disclosed.

In a related aspect, the composition is combined with one or more meat substitutes. In a further related aspect, the meat substitute includes thawed and sliced frozen tofu, oncom, tempeh, tofu, tofurkey, faux turkey, paneer, glamorgan, breadfruit, sapal, eggplant, jackfruit, falafel, ganmodoki, and combinations thereof. In a further related aspect, the concentrate improves one or more of the sensory characteristics including texture, aroma, mouthfeel, bite, crunch, flavor, appearance, or combinations thereof, of said one or more meat substitutes compared to the same meat substitutes lacking said concentrate. In another related aspect, the foodstuff is for human consumption.

In one aspect, the feed is formulated for animals including fin fish, shell fish, crustaceans, domestic animals, farm animals, and combinations thereof.

In another aspect, the *A. pullulans* is NRRL-Y-2311-1.

In one aspect, there is a significant shift downward in raw NIR spectra between 4664 cm-1 and 4836 cm-1 for the final product relative to the feed stock. In a related aspect, the shift downward is between about at least 10% to about 20%.

In embodiments, a method of treating plant-based material is disclosed including: a) transferring the plant-based material to a first mix-tank, where the plant-based material is mixed with one or more first solvents to produce a washed mash; b) separating the washed mash into at least one centrate and a washed cake; c) transferring the washed cake to one or more second mix-tanks, where one or more second solvents are mixed with the washed cake to produce a washed cake suspension; d) transferring the washed cake suspension to one or more fermenters, where the transferred washed cake suspension is inoculated with at least one microbe, and where the inoculated washed cake suspension is incubated for a sufficient time to produce a fermented mixture; e) heating the fermented mixture for a time sufficient to achieve a degree of hydrolysis (DH) of between about 2% to about 80% of the proteins therein; f) separating the heated fermented mixture into a fermented centrate and a fermented cake; g) transferring the fermented centrate to:

(i) a first mix-tank and/or

(ii) one or more second mix-tanks,

where a mix-tank comprises the plant-based material or the washed cake, and where steps (c)-(f) and h) are repeated at least one (1) time for sub-steps (i) or (ii); and h) drying the fermented cake, where the at least one microbe does not generate sufficient exopolysaccharides to produce a viscous fermented cake during drying, and where the resulting dried fermented cake has a higher protein content and/or has substantially decreased antinutritional factors compared to the transferred plant-based material.

In one aspect, the at least one microbe produces less than about 3g/L of pullulan when grown in a medium comprising between 0.35 and 0.5 g/L yeast extract.

In another aspect, the method further includes transferring the at least one centrate of step (b) to one or more of the mix-tanks prior to inoculation.

In one aspect, recycling of the centrates: a) reduces the amount of fresh solvent added to a first mix-tank and/or one or more second mix-tanks and/or b) increases yield and recovery of proteinaceous materials.

In another aspect, the method does not include addition of cellulose deconstructing enzymes.

In one aspect, the method further includes heating the washed cake suspension prior to transfer to one or more fermenters. In a related aspect, the washed cake suspension is heated to greater than 100°C.

In another aspect, the fermentation centrate is transferred to the first mix tank.

In a related aspect, the centrates and cakes are produced by hydrodynamic force, and where the method includes a system of four (4) mix tanks and four (4) centrifuges in series, where the fermentation centrate of mix-tank 4 is transferred to mix-tank 3, the centrate of mix-tank 3 is transferred to mix-tank 2, and the centrate of mix-tank 2 is transferred to mix-tank 1 prior to a second fermentation.

In one aspect, the solvent includes water, an acid, an aqueous enzyme mixture, antifomates or a combination thereof, where the aqueous enzyme mixture comprises phytase.

In another aspect, the centrate from the first mix tank, the fermented centrate, or both are transferred to at least one evaporator producing a liquid protein condensate. In a related aspect, the centrate is evaporated at a temperature of between about 60°C to 90°C and/or about 1 psia to 6 psia.

In one aspect, the non-animal based protein concentrate is isolated from plant material including soybeans, sorghum, peanuts, pulses, Rapeseeds, oats, barley, rye, lupins, fava beans, canola, peas, sesame seeds, cottonseeds, palm kernels, barley, grape seeds, olives, safflowers, sunflowers, copra, corn, coconuts, linseed, hazelnuts, wheat, rice, potatoes, cassavas, legumes, camelina seeds, mustard seeds, germ meal, corn gluten meal, distillery/brewery by-products, and combinations thereof.

In another aspect, drying is carried out at greater than 100°C, and where the dried fermented cake exhibits a moisture content of less than about 7%.

In one aspect, the at least one microbe is NRRL Y-2311-1. In another aspect, the microbe may be identified by targeting for the presence of amplification products from SEQ ID NO:1 as the template via PCR. In a related aspect, the amplification products may be used to identify the source of the HQPC as disclosed herein.

In one aspect, there is a significant shift downward in raw NIR spectra between 4664 cm-1 and 4836 cm-1 for the final product relative to the feed stock. In a related aspect, the shift downward is between about at least 10% to about 20%.

In another aspect, treating does not include adding one or more cellulose-deconstructing enzymes.

In embodiments, a composition is disclosed including a solid protein concentrate produced by the methods described above.

In embodiments, a feed or foodstuff is disclosed including the compositions described above.

In a related aspect, the feed is formulated for animals including fin fish, shell fish, crustaceans, domestic animals, farm animals, and combinations thereof. In a further related aspect, the composition is for human consumption.

In embodiments, a method of improving the survival of juvenile shrimp is disclosed including feeding juvenile shrimp with the feeds as described above, where the degree of hydrolysis (DH) of the protein in the feed by shrimp enzymes is at least 7%. In a related aspect, the predicted apparent protein digestibility (PPD) of the feed is at least 90%.

In embodiments, a feed for juvenile shrimp is disclosed including the composition as described above, where the feed exhibits a degree of hydrolysis (DH) of at least 7%, a predicted apparent protein digestibility (PPD) of at least 90%, or a combination thereof.

## BRIEF DESCRIPTION OF THE DRAWINGS

Figure 1 shows a flow chart for an HQSPC conversion process.

Figure 2 shows a flow chart for an HQSPC conversion process for aqua feeds.

Figure 3 shows bench scale, extended incubation trials to evaluate HQSPC composition and yield.

Figure 4 shows a flow chart for an HP-DDGS conversion process for aqua feeds.

Figure 5 shows the effect of moisture content and extrusion speed on glucose recovery following extrusion of HP-DDGS at 100° C.

Figure 6 shows an outline of a separate HQPC process as disclosed herein.

Figure 7 shows details for centrate 1.

Figure 8 shows details for centrate 4.

Figure 9 shows value added products for outline in Figure 6.

FIG. 10 shows raw spectra for different SBM feed stock samples. The x-axis is wavenumber and the y-axis is intensity in absorbance units (AU).

FIG. 11 shows raw spectra for different HQPC products made from the SBM samples in FIG. 10. The x-axis is wavenumber and the y-axis is intensity in AU.

FIG. 12: Final weight (g) of Rainbow trout fed diets containing HQPC (25%) vs. fish meal diets.

FIG. 13a: Weekly growth rate (g) performance of Coho Salmon fed HQPC.

FIG. 13b: Feed conversion rate (FCR) of Coho Salmon fed HQPC.

FIG. 14: Survival of first feeding *L. vannamei* larvae (Z3 -PL13) fed artificial feeds containing HQPC.

FIG. 15: Average Body Weight (g) of *L. vannamei* juveniles fed artificial feeds containing HQPC.

FIG. 16: Survival rate (%) of *L. vannamei* juveniles fed artificial feeds containing HQPC on day 10th of a post-challenge with EMS. Means sharing the same superscript are not significantly different from each other (Tukey's HSD, $P<0.05$).

## DETAILED DESCRIPTION OF THE INVENTION

Before the present composition, methods, and methodologies are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "a formulation" includes one or more formulations, and/or compositions of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Any methods and materials similar or equivalent to those described herein can be used in the practice or testing of the invention, as it will be understood that modifications and variations are encompassed within the spirit and scope of the instant disclosure.

As used herein, "about," "approximately," "substantially" and "significantly" will be understood by a person of ordinary skill in the art and will vary in some extent depending on the context in which they are used. If there are uses of the term which are not clear to persons of ordinary skill in the art given the context in which it is used, "about" and "approximately" will mean plus or minus <10% of the particular term and "substantially" and "significantly" will mean plus or minus >10% of the particular term.

Internal studies developing practical diets for RAS operations using a microbial enhanced protein as described, have shown the HQPC as disclosed herein to be a promising solution for the production of eco-friendly aquaculture feeds. Besides having over 70 percent crude protein content and highly available phosphorus content, the HQPC as disclosed herein may be manufactured from non-GM soybeans, which makes it a perfect match for the European aqua industry.

Results from numerous internal feeding trials have demonstrated that HQPC as disclosed herein can sustain fish and shrimp health, high-performance growth and feed efficiency with inclusion levels at super-dose levels as high as 70% of the total amount of ingredients in the diet. Feeding trials using Rainbow trout, Barramundi and Coho salmon have shown that fish fed HQPC based feeds as disclosed consistently utilized feed more efficiently than the fish fed the control feed. Trials conducted using those species reared in a RAS system displayed good growth rates when fed diets containing HQPC as disclosed at levels up to 25% and reduced feed conversion rates.

A central problem that confronts sustainable RAS involves two interlocking limitations that the operators must balance to run successful aquaculture operations. The first limitation involves an understanding of present-day water re-use systems that limit fish or shrimp production. The second limitation centers on sustainable fish feeds where use of soy-based feeds in such present-day water re-uses systems make their management more difficult. The resulting situation requires present day aquaculture farmers to balance the risk-benefit of each limitation so as to achieve profitability as well as fulfil the demands of their customers.

Formulating low fish meal aquaculture feeds for RAS systems requires the use of combinations of several ingredients since most feedstuffs have been shown to have significant nutrient and functional limitations. Fermentation of plant ingredients can reduce the anti-nutritional factors and enhance the digestibility. While not being bound by theory, the significant differences observed in the present disclosure with growth performance of fish and shrimp fed HQPC is probably due to the elimination of anti-nutritional factors and increased digestibility of the ingredient. The results as shown herein indicate that at least 80% of the dietary fish meal can be directly replaced by an HQPC as described in commercial feeds for, *inter alia,* shrimp larvae, and several juvenile and adult fish species.

As used herein, the term "animal" means any organism belonging to the kingdom Animalia and includes, without limitation, humans, birds (e.g., poultry), mammals (e.g., humans, cattle, swine, goat, sheep, cat, dog, mouse and horse) as well as aquaculture organisms such as fish (e.g., trout, salmon, perch), mollusks (e.g., clams) and crustaceans (e.g., crab, lobster, prawns and shrimp).

Use of the term "fish" includes all vertebrate fish, which may be bony or cartilaginous fish.

As used herein "non-animal based protein" means that the substance comprises at least 0.81 g of crude fiber/100g of composition (dry matter basis), which crude fiber is chiefly cellulose and lignin material obtained as a residue in the chemical analysis of vegetable substances.

As used herein, "incubation process" means the provision of proper conditions for growth and development of bacteria or cells, where such bacteria or cells use biosynthetic pathways to metabolize various feed stocks. In embodiments, the incubation process may be carried out, for example, under aerobic conditions. In other embodiments, the incubation process may include fermentation.

As used herein, the term "incubation products" means any residual substances directly resulting from an incubation process/reaction. In some instances, an incubation product contains microorganisms such that it has a nutritional content enhanced as compared to an incubation product that is deficient in such microorganisms. The incubation products may contain suitable constituent(s) from an incubation broth. For example, the incubation products may include dissolved and/or suspended constituents from an incubation broth. The suspended constituents may include undissolved soluble constituents (e.g., where the solution is supersaturated with one or more components such as proteins) and/or insoluble materials present in the incubation broth. The incubation products may include substantially all of the dry solids present at the end of an incubation (e.g., by spray drying an incubation broth and the biomass produced by the incubation) or may include a portion thereof. The incubation products may include crude material from incubation where a microorganism/solids/centrates/cakes may be fractionated and/or partially purified to increase the nutrient content of the material.

As used herein, a "conversion culture" means a culture of microorganisms which are contained in a medium that comprises material sufficient for the growth of the microorganisms, e.g., water and nutrients. The term "nutrient" means any substance with nutritional value. It can be part of an animal feed, foodstuff or food supplement for an animal. Exemplary nutrients include but are not limited to proteins, peptides, fats, fatty acids, nucleic acids, lipids, water and fat soluble vitamins, essential amino acids, carbohydrates, sterols, enzymes and trace minerals, such as, phosphorus, iron, copper, zinc, manganese, magnesium, cobalt, iodine, selenium, molybdenum, nickel, fluorine, vanadium, tin, silicon and combinations thereof.

Conversion is the process of culturing microorganisms in a conversion culture under conditions suitable to convert protein/carbohydrate/polysaccharide materials, for example, soybean constituents into a high-quality protein concentrate. Adequate conversion includes, but is not limited to, utilization of 90% or more of specified carbohydrates to produce microbial cell mass and/or exopolysaccharide, enzymes and microbial metabolites, specific reduction in oligosaccharide concentration, achieving a select degree of hydrolysis for proteins, seeing a specific %change in NIR spectra between 4664 $cm^{-1}$ and 4836 $cm^{-1}$ or a combination thereof. In embodiments, conversion may be aerobic or anaerobic or a combination thereof.

As used herein a "flocculent" or "clearing agent" is a chemical that promotes colloids to come out of suspension through aggregation, and includes, but is not limited to, a multivalent ion and polymer. In embodiments, such a flocculent/clearing agent may include bioflocculents such as exopolysaccharides (e.g., pullulan).

As used herein, "formulation" means a material or mixture prepared according to a particular prescription.

As used herein, "foodstuff" means a substance suitable for consumption as a nutritious composition that humans or animals eat or drink or that plants absorb in order to maintain life and growth.

As used herein, "centrate" means the liquid leaving a hydrodynamic force application after most of the solids have been removed, the resulting dry product is termed "cake".

As used herein, "suspension" means a heterogeneous mixture that contains solid particles sufficiently large for sedimentation.

As used herein, "evaporation" means the process of turning from liquid into vapor. The main difference between evaporation and distillation is that evaporation is a process that involves a change in the state of matter while distillation is a process of separation. Both these processes may be used for various purposes. While the vaporization in evaporation occurs below the boiling point, the vaporization in distillation occurs at the boiling point.

As used herein, "exopolysaccharides" means high-molecular-weight polymers comprising sugar residues that are generated by a microorganism and released into the surrounding environment, which high-molecular weight polymers include anabolic and metabolic products.

As used herein, "degree of hydrolysis (DH)", means the proportion of cleaved peptide bonds in a protein hydrolysate. Several methods exist for determining DH; the most commonly used of these include the pH-stat, trinitrobenzenesulfonic acid (TNBS), o-phthaldialdehyde (OPA), trichloroacetic acid soluble nitrogen (SN-TCA), and formal titration methods.

As used herein, "trypsin inhibitor unit (TIU)" means the amount of trypsin inhibitor in a sample. For example, a method may use N-benzoyl-DL-arginine p-nitroanilide as a chromogenic substrate for trypsin, and the ability of aliquots of soy meal extract to inhibit the activity of trypsin towards this substrate is utilized to estimate the amount of trypsin inhibitor in a soy meal sample. The amount of *p*-nitroaniline formed during a 10-min incubation is measured spectrophotometrically, and the absorbance values in the presence and absence of soy extract are used in calculations that give a number for trypsin inhibitor units (TIU) per gram of original soy sample.

As used herein, "meat substitute" or "meat analog" means a composition that approximates certain aesthetic qualities (primarily texture, flavor and appearance) or chemical characteristics of a specific meat. In embodiments, such substitutes or analogs include, but are not limited to, dairy-based: paneer cheese, glamorgan sausage, paneer; fungi-derived: edible mushrooms, mycoprotein, fistulina hepatica, lyophyllum decastes; fruit-based: tempeh, breadfruit, coconut burger, green jackfruit pulp, eggplant, jackfruit; legumes: Burmese tofu, falafel, ganmodoki, koya-dofu, oncom (red oncom and black oncom), tempeh burger, textured vegetable protein, tofurkey or faux turkey, vegetarian bacon, vegetarian hot dog, vegetarian sausage, and veggie burger. In one aspect, the protein concentrate as disclosed herein is combined with meat substitutes.

As used herein, "NIR (near-infrared spectroscopy)" is a non-invasive detection method for determining protein content.

As used herein, "hydrodynamic force" means energy acting on solid bodies immersed in fluids and in motion relative to said fluids. In a related aspect, such force may be applied through processes, including, but not limited to, centrifugation and filtration.

As used herein, "cellulose deconstructing enzymes" means enzymes that act by hydrolyzing, *inter alia,* glycosidic bonds of linear glucose β-1,4-linked polymers, producing glucose and other simple or complex sugars.

As used herein, "antifomate" or "defoamer", including grammatical variations thereof, is a chemical additive that reduces and hinders the formation of foam in industrial process liquids. In a related aspect, such chemicals include, but are not limited to, oil based defoamers; powder defoamers; water based defoamers, silicone based defoamers; EO/PO based defoamers and alkyl polyacrylates. In a related aspect, such a defoamer includes water-based food-grade emulsion designed to control foam in aqueous food-canning processes, non-aqueous silicone-free defoamer that utilizes defoaming polymers and biodegradable oils, and food-grade 100% active food-grade kosher defoamer designed to destroy foam in aqueous environments including food manufacturing, fermentation, agricultural and industrial-grade processes.

As used herein, "predicted apparent protein digestibility (PPD)" is the measure of a regression calculation between in vivo apparent protein digestibility (APD) and in vitro protein digestion with digestive enzymes (e.g., degree of hydrolysis) of different feed ingredients.

As used herein, "room temperature" is about 25° C under standard pressure.

As used herein, "APD" is a measure of the ratio of the difference of the ingested and fecal nitrogen to the ingested nitrogen, expressed as a percentage.

As used herein, "HQPC" means high quality protein concentrate from one or more fermented plant-based materials. Such HQPC may be used as a feed, feedstock, alone or in combination with other feed or feedstock constituents, a pro-biotic, or a constituent thereof, including as a means to deliver nutraceuticals and/or pharmaceuticals to animals. In embodiments, the protein content of the HQPC may be about 60% to about 65%, about 65% to about 70%, about 70% to about 75% or greater (dry matter basis (dmb)).

As used herein, "solvent" means a substance, ordinarily a liquid, in which other materials dissolve to form a solution. Polar solvents (e.g., water, aqueous solutions) favor formation of ions; nonpolar solvents (e.g., hydrocarbons) do not. Solvents may be predominantly acidic, predominantly basic, amphoteric, or aprotic. Organic compounds used as solvents include, but are not limited to, aromatic compounds and other hydrocarbons, alcohols, esters, ethers, ketones, amines, and nitrated and halogenated hydrocarbons.

Plant Protein Sources

A large number of plant protein sources may be used in connection with the present disclosure as feed stocks for conversion. The main reason for using plant proteins in the feed industry is to replace more expensive protein sources, like animal protein sources. Another important factor is the danger of transmitting diseases through feeding animal proteins to animals of the same species.

Examples for plant protein sources include, but are not limited to, protein from the plant family Fabaceae as exemplified by soybean and peanut, from the plant family Brassiciaceae as exemplified by canola, cottonseed, the plant family Asteraceae including, but not limited to sunflower, and the plant family Arecaceae including copra. These protein sources, also commonly defined as oilseed proteins may be fed whole, but they are more commonly fed as a by-product after oils have been removed. Other plant protein sources include plant protein sources from the family Poaceae, also known as Gramineae, like cereals and grains especially corn, wheat and rice or other staple crops such as potato, cassava, and legumes (peas and beans), some milling by-products including germ meal or corn gluten meal, or distillery/brewery by-products. In embodiments, feed stocks for proteins include, but are not limited to, plant materials from soybeans, sorghum, peanuts, pulses, Rapeseeds, oats, barley, rye, canola, sesame seeds, cottonseeds, palm kernels, grape seeds, olives, safflowers, sunflowers, copra, corn, coconuts, linseed, hazelnuts, wheat, rice, potatoes, cassavas, legumes, pennycress seeds, camelina seeds, mustard seeds, wheat germ meal, corn gluten meal, corn gluten feed, distillery/brewery by-products, and combinations thereof.

In the farming industry the major proteins of plant origin reportedly used, include, but are not limited to, soybean meal (SBM), maize gluten meal, Rapeseed/canola (Brassica sp.) meal, lupin (Lupinus sp.), for example, the proteins in kernel meals of de-hulled white (Lupinus albus), sweet (L. angustifolius) and yellow (L. luteus) lupins, Sunflower (Helianthus annuus) seed meal, crystalline amino acids; as well as pea meal (Pisum sativum), Cottonseed (Gossypium sp.) meal, Lemnoidae (duckweed or water lentils), Peanut (groundnut; Arachis hypogaea) meal and oilcake, soybean protein concentrate (SPC), soy protein isolate (SPI), corn (Zea mays) gluten meal and wheat (Triticum aestivum) gluten, Potato (Solanum tuberosum L.) protein concentrate as well as other plant feedstuffs like Moringa (Moringa oleifera Lam.)

leaves, all in various concentrations and combinations.

The protein sources may be in the form of non-treated plant materials and treated and/or extracted plant proteins. As an example, heat treated soy products have high protein digestibility.

A protein material includes any type of protein or peptide. In embodiments, soybean material or the like may be used such as whole soybeans. Whole soybeans may be standard, commoditized soybeans; soybeans that have been genetically modified (GM) in some manner; or non-GM identity preserved (IP) soybeans. Exemplary GM soybeans include, for example, soybeans engineered to produce carbohydrates other than stachyose and raffinose. Exemplary non-GM soybeans include, for example, Schillinger varieties that are line bred for low carbohydrates, and low trypsin inhibition. High protein varieties include, but are not limited to, N2358 (Benson Hill Inc., St. Louis, MO).

Other types of soybean material include soy protein flour, soy protein concentrate, soybean meal and soy protein isolate, or mixtures thereof. The traditional processing of whole soybean into other forms of soy protein such as soy protein flours, soy protein concentrates, soybean meal and soy protein isolates, includes cracking the cleaned, raw whole soybean into several pieces, typically six (6) to eight (8), to produce soy chips and hulls, which are then removed. Soy chips are then conditioned at about 60° C and flaked to about 0.25 millimeter thickness. The resulting flakes are then extracted with an inert solvent, such as a hydrocarbon solvent, typically hexane, in one of several types of countercurrent extraction systems to remove the soybean oil. For soy protein flours, soy protein concentrates, and soy protein isolates, it is important that the flakes be desolventized in a manner which minimizes the amount of cooking or toasting of the soy protein to preserve a high content of water-soluble soy protein. This is typically accomplished by using vapour desolventizers or flash desolventizers. The flakes resulting from this process are generally referred to as "edible defatted flakes" or "white soy(bean) flakes."

White soy bean flakes, which are the starting material for soy protein flour, soy protein concentrate, and soy protein isolate, have a protein content of approximately 50%. White soybean flakes are then milled, usually in an open-loop grinding system, by a hammer mill, classifier mill, roller mill or impact pin mill first into grits, and with additional grinding, into soy flours with desired particle sizes. Screening is typically used to size the product to uniform particle size ranges, and can be accomplished with shaker screens or cylindrical centrifugal screeners. Other oil seeds may be processed in a similar manner.

Soybeans contain a small but very significant 2S albumin storage protein, in addition to glycinin and beta-conglycinin. Soybeans also contain biologically active or metabolic proteins, such as enzymes, trypsin inhibitors, hemagglutinins, and cysteine proteases very similar to papain.

While soy products have high protein digestibility, the upper inclusion level for full fat or defatted soy meal inclusion in diets for carnivorous fish, for example, is between an inclusion level of 20 to 30%, even if heat labile antinutrients are eliminated. In fish, soybean protein has shown that feeding fish with protein concentration inclusion levels over 30% causes intestinal damage and in general reduces growth performance in different fish species. In fact, most farmers are reluctant to use more than 10% plant proteins in the total diet due to these effects.

The present invention solves this problem and allows for plant protein inclusion levels of up to 40 or even 50%, depending on, amongst other factors, the animal species being fed, the origin of the plant protein source, the ratio of different plant protein sources, the protein concentration and the amount, origin, molecular structure and concentration of the glucan and/or mannan. In embodiments, the plant protein inclusion levels are up to 40%, preferably up to 20 or 30%. Typically, the plant protein present in the diet is between 5 and 40%, preferably between 10 or 15 and 30%. These percentages define the percentage amount of a total plant protein source in the animal feed or diet, this includes fat, ashes etc. In embodiments, pure protein levels are up to 50%, typically up to 45%, in embodiments 5-95%.

The proportion of plant protein to other protein in the total feed or diet may be 5:95 to 95:5, 15:85 to 50:50, or 25:75 to 45:55.

In addition to feed diets, HQPC as disclosed herein may be formulated so as to be used with meat substitutes. In embodiments, HQPC may be combined with fermented soy-based products. Examples of fermented soy-based products include, but are not limited to, thawed and sliced frozen tofu; oncom, one of the traditional staple foods of West Java (Sundanese) cuisine of Indonesia, there are two types: red oncom and black oncom (oncom is closely related to tempeh; both are foods fermented using mold); soy protein; soy pulp, used for veggie burgers and croquettes); tempeh, a traditional Indonesian soy product made from fermented soybeans; textured vegetable protein, a defatted soy flour product that is a by-product of extracting soybean oil (it is often used as a meat analogue or meat extender, with a protein content that is comparable to certain meats); tofu (while not traditionally seen as a meat substitute in Asia, but widely used for that purpose in the Western hemisphere); and tofurkey, faux turkey, a meat substitute in the form of a loaf or casserole of vegetarian protein, usually made from tofu (soybean protein) or seitan (wheat protein) with a stuffing made from grains or bread, flavored with a broth and seasoned with herbs and spices; paneer cheese; glamorgan sausage; mushrooms, including but not limited to, Fistulina hepatica, Laetiporus, and Lyophyllum decastes; breadfruit; coconut burger (made from sapal, the coconut pulp by-products of traditional coconut milk extraction); eggplant; jackfruit; falafel; and ganmodoki.

In embodiments, the HQPC-combined fermented soy product as disclosed, may be used as a meat substitute alone, or may be combined with other meat substitutes or meat analogs to produce various products for human consumption, including various combinations comprising the examples above. In one aspect, the addition of the HQPC is combined with various meat substitutes and/or meat analogues to improve texture, aroma, feel, bite, crunch, flavor or appearance of said meat substitute and/or meat analogue. Such aesthetics may be determined, for example, using Caswell's classification of food quality (see, e.g., Caswell, J Agr Res Econ (1998) 42:409-474).

Microorganisms

The disclosed microorganisms must be capable of converting carbohydrates and other nutrients into a high-quality protein concentrate in the process as disclosed herein. In embodiments, the microorganism is a yeast-like fungus. An example of a yeast-like fungus is *Aurobasidium pullulans.* Other example microorganisms include yeast such as *Kluyveromyces* and *Pichia* spp, Lactic acid bacteria, *Trichoderma reesei, Pleurotus ostreatus, Rhizopus* spp, and many types of lignocellulose degrading microbes. Generally, exemplary microbes include those microbes that can metabolize stachyose, raffinose, starch, glucose, fructose, lactose, sucrose, xylose and other sugars. However, it is within the abilities of a skilled artisan to pick, without undue experimentation, other appropriate microorganisms based on the disclosed methods.

In embodiments, microorganisms exhibit low exopolysaccharide (e.g., pullulan) production, for example, low pullulan yield is considered less than about 3.0 g/L when grown in yeast extract (YE) containing media, where YE (as nitrogen source) is present between 0.35 and 0.5 g/L (see, e.g., Leathers et al., J Indus Micro (1988) 3:231-239; at p. 232, col. 2, third paragraph, and Table 3, incorporated herein by reference). While not being bound by theory, high exopolysaccharide producers generate final products that can be difficult to dry (e.g., extends drying time) and/or generate viscous products after drying. Methods for determining pullulan content may be used as disclosed in Leathers et al. ((1988), at p. 232 col. 1, third paragraph bridging to col. 2, first paragraph; herein incorporated by reference), however, the skilled artisan would recognize that alternative methods are available.

In embodiments, the *A. pullulans* is adapted to various environments/stressors encountered during conversion. In one aspect, the *A. pullulans* strain is selected from NRRL deposit Nos. Y-2311-1, Y-6754a, YB-4026, YB-4588, Y-6992, Y-17000, or Y-17001, and combinations thereof. In a related aspect, an *A. pullulans* strain denoted by NRRL deposit No. Y-2311-1 may be used as disclosed herein.

In a related aspect, the microorganism may be identified using PCR. In embodiments, the organism may be targeted by directing primers to the following nucleic acid sequence encoding alpha-arabinofuranosidase (Genbank Accession No.: AY495375):

(SEQ ID NO: 1):

```
  1 gatcccgccg gattacggaa aataacagag cgagttcgta tgcgatgatc ttcgctggag  61
    atgtgctaca tccacagctc gaacataaat agagaagaca atgccgcctg gctgtccaac 121
    atcaactcct ctcatatccg caagcttcct gtcaaccctc ctcacagttc gctcatcact 181
    caaacatgcg ttccaggacg aacatcgctc ttggcctagc tgccactggt tccctagtcg 241
    ctgccgcgcc ttgcgatatc tatcagaatg gcggtactcc ttgcgtagct gctcacggca 301
    caactcgcgc attgtatgat tcctacactg gtcctctcta ccaacttaag agaggctcag 361
    atggcactac gaccgatatt tctcctttgt ctgctggtgg tgttgccaat gctgctgctc 421
    aggactcttt ctgcaagggt actacctgtc ttatcagtat tatctacgat cagtctgggc 481
    gtgcaaacca tctttatcag gcccagaaag gtgctttcag cggaccagat gtcaacggaa 541
    acgacaactt ggcaggcgct attggagcac cagtgacttt gaatggcaag aaggcatatg 601
    gcgtgttcat ctcgcccggc actgggtaca gaaacgacga agtcagcggc acggccactg 661
    gaaacgaacc tgagggcatg tatgctgttc ttgacggcac tcattacaac gatgcttgct 721
    gctttgacta cggaaacgcg gaaatcagca acacggatac tggtaacgga catatggagg 781
    ccgtctacta tggtaacaac acgatttggg gcagtggctc tggcagcggt ccttggctca 841
    tggccgacct tgagaacggt ttgttctctg gccagggtac caagcagaac actgcagacc 901
    cttcaatctc caacagattc ttcaccggaa tggtcaaggg agagcctaac cagtgggcgc 961
```

```
ttcgcggtag caatgccgcg tccggttcct tgtcgaccta ctacagtggc gctcgtccca 1021
ccgtcggcgg ttacaacccc atgagcctcg agggcgccat cattcttggc atcggtggcg 1081
ataacagcaa tggcgctcag ggcactttct atgagggggt catgacctcg ggctacccgt 1141
ctgatgccac tgaagcctcg gtgcaggcca acattgtggc tgcgaagtac gctaccacat 1201
ctttgaacac agcaccactc actgtcggca acaagatttc gatcaaggtg accacccccg 1261
gctacgacac ccgctatctg gcacacaccg gagccaccgt caacacgcag gttgtctctt 1321
catctagcgc gactagcctc aagcagcagg ccagctggac tgttcgcaca ggcctcggta 1381
acagcggctg ttactctttc gagtcggttg atacacctgg aagcttcatc agacactaca 1441
acttccagct ccagctcaac gcgaatgaca acaccaaggc tttcaaggaa gacgcgactt 1501
tctgctctca gaccggtctt gttaccggca acactttcaa ctcgtggagc taccctgcca 1561
agttcatccg tcactacaac aatgttggat acatcgccag caacggtggt gttcacgact 1621
ttgactctgc tacaggcttc aacaacgatg tcagctttgt ggttggaagc agctttgctt 1681
agatgtaaaa ggtcaggatg aatatgatgg atgtttatga caaaagaagt tatgagtttg 1741
tagttatgga atcttagctg tagcttttga aagcctttgg gatatcagat gtttgtctct 1801
tgttcatgtg ccgttgcaaa gaagaaaaga aggagcagca agcagtgagg ctcttatcgg 1861
gcgatagggc tagatc
```

In a related aspect, the following primers may be used:

| | |
|---|---|
| Forward 1: 5' TGACTACGGAAACGCGGAAA 3' | SEQ ID NO: 2 |
| Reverse 1: 5' CATTGCTGTTATCGCCACCG 3' | SEQ ID NO: 3 |
| Forward 2: 5' GCTTCCTGTCAACCCTCCTC 3' | SEQ ID NO: 4 |
| Reverse 2: 5' CACGCCATATGCCTTCTTGC 3' | SEQ ID NO: 5 |
| Forward 3: 5' TTGACTACGGAAACGCGGAA 3' | SEQ ID NO: 6 |
| Reverse 3: 5' AGGCTTCAGTGGCATCAGAC 3' | SEQ ID NO: 7 |
| Forward 4: 5' TCAGCGGACCAGATGTCAAC 3' | SEQ ID NO: 8 |
| Reverse 4: 5' TTTCCGCGTTTCCGTAGTCA 3' | SEQ ID NO: 9 |
| Forward 5: 5' CAACACGATTTGGGGCAGTG 3' | SEQ ID NO: 10 |
| Reverse 5: 5' GAGCGCCATTGCTGTTATCG 3' | SEQ ID NO: 11 |
| Forward 6: 5' CGGAAACGACAACTTGGCAG 3' | SEQ ID NO: 12 |
| Reverse 6: 5' GTGTTGCTGATTTCCGCGTT 3' | SEQ ID NO: 13 |
| Forward 7: 5' CGATAACAGCAATGGCGCTC 3' | SEQ ID NO: 14 |
| Reverse 7: 5' GAGGCTAGTCGCGCTAGATG 3' | SEQ ID NO: 15 |
| Forward 8: 5' GGAATGGTCAAGGGAGAGCC 3' | SEQ ID NO: 16 |
| Reverse 8: 5' GAGCGCCACTGTAGTAGGTC 3' | SEQ ID NO: 17 |
| Forward 9: 5' CCGGCAACACTTTCAACTCG 3' | SEQ ID NO: 18 |
| Reverse 9: 5' CCTATCGCCCGATAAGAGCC 3' | SEQ ID NO: 19 |
| Forward 10: 5' CACTGGTTCCCTAGTCGCTG 3' | SEQ ID NO: 20 |
| Reverse 10: 5' GGCTCTCCCTTGACCATTCC 3' | SEQ ID NO: 21 |

In one aspect, the primer pairs may be used alone or in combination with each other or other primer pairs. In another aspect, the primer pairs may be used to track and identify origin of products made by the methods described herein. PCR may be carried out by standard methods (see, e.g., U.S. Pat. No. 4,800,159, herein incorporated by reference), although alternative PCR methods would be apparent to the skilled artisan.

While not being bound by theory, it has been observed that the viscosity of the final product(s) seem to be dependent on novel materials made as a consequence of fermentation, e.g., exopolysaccharides that would only exist where the plant material contains a substrate that is metabolized by the microorganism that produce a high-viscosity producing, plant based substrate, fermentation dependent product (novel-viscosity increasing exopolysaccharide) that is not pullulan. As such, a low exopolysaccharide/pullulan producing organism may be alternatively replaced by using an organism that does not produce the novel-viscosity increasing exopolysaccharide.

Production Process

In exemplary embodiments, after optional pretreatment (e.g., to increase availability of nutrients to cells, remove sugars and the like), the plant-based material may be blended with water and/or centrate to form a mash in one or more mix tanks at a solid loading rate of at least 5%, with pH adjusted to 4.5-4.9. In one aspect, the pH is 4.8. The mash may be treated with sulfuric acid and/or defoamers prior to application of hydrodynamic force to separate the suspension into a cake and centrate. The cake may be further washed with one or more solvents (e.g., water or centrate) before transfer to a cook tube-cooler tandem device(s), where the residence time in the cook-tube may be varied. Temperature in the cook tube may be varied up to 121.1° C, and residence time may be varied from 0 up to 2 min. In one aspect, the residence time is about 1.5 mins.

After cooling to about 30° C, the cooled mash is transferred to one or more fermenter vessels, and an inoculum of *A. pullulans* may be added to the mash, where the resulting inoculated mash may be incubated for about 7h to about 10 h, about 10 h to about 14h, about 14 h to about 20 h, or about 7 h to about 24 h, or until the degree of hydrolysis (DH) of 2% to 80% of protein is achieved. While not being bound by theory, DH is substantially due to enzyme hydrolysis rather than pyrolysis, including that DH may be greater with additional recycling of centrate from broth tank. Inoculation volume (e.g., from a 60hr seed culture, approximately between about $1 \times 10^1$ to about $100 \times 10^9$ CFU/ml) may be about 1% of the working volume of the one or more fermenter vessels. In a related aspect, inoculated cells may have substantially unicellular morphology, and while not being bound by theory, use of cells with substantially filamentous morphology may result in decreased access to oxygen and other nutrients. During incubation, sterile air may be introduced into the reactor at a rate of 0.5-1 L/L/h. In embodiments, the conversion culture undergoes conversion by incubation with the plant-based material for less than about 12 hours. In embodiments, the conversion culture will be incubated for between about 7 hours and about 14 hours. The conversion culture may be incubated at about 24-35° C.

In embodiments, the pH of the conversion culture, while undergoing fermentation, may be about 4.5 to about 5.5. In embodiments, the pH of the conversion culture may be about 4.8. In embodiments, the conversion culture is actively aerated.

The fermented plant material is transferred from one or more incubating vessels to a broth tank, where it may be heated to about 60° C, from about 30 mins to 2 hr. Hydrodynamic force is applied to the heated-fermented plant material, which results in a fermented cake and fermented centrate. The fermented cake is then dried to less than about 7% moisture, at a temperature of between about 37.8° C to about 149° C. The fermented centrate may be transferred to one or more of the previously employed mix tanks (e.g., to conserve and recycle water, increase yield and protein content, including soluble proteins), to be mixed with incoming plant-based material, including that the fermented centrate may be transferred to an evaporator to produce a liquid protein concentrate.

Fermented centrate destined for the evaporator may be subject to two or more evaporation steps, where evaporation is carried out for a sufficient time and temperature to achieve a liquid protein concentrate having % solids from about 10 to about 60%. Alternatively, the liquid protein concentrate may be transferred back to one or more of the mix tanks to ultimately form one or more additional centrates/cakes for processing. In one aspect, the cakes/centrates may be washed and/or precipitated with ethanol.

In embodiments, final protein concentrations solids recovery may be modulated by plant-based feedstock, incubation conditions, pH, drying time and temperature. For example, about 70% protein or greater may be achieved for the ultimate dried cake with a 14 hr incubation, where the solids content of between about 76.59% to about 99.65% is obtained. Protein content in said cake may be from about 65% to about 70%, about 70% to about 75%, or about 75% to about 80% (dmb).

In embodiments, the feed stock may be treated with one or more antibiotics (e.g., but not limited to, tetracycline, penicillin, erythromycin, tylosin, virginiamycin, and combinations thereof) before inoculation with the converting microbe to avoid, for example, contamination by unwanted bacteria strains.

During incubation, samples may be removed at regular intervals during processing (e.g., determine amino acids, DH, oligosaccharide concentration, pullulan content and the like). For example, samples for HPLC analysis may be boiled, centrifuged, filtered (e.g., through 0.22-μm filters), placed into autosampler vials, and frozen until analysis. In embodiments, samples may be assayed for carbohydrates and organic solvents using a WATERS HPLC system, although other HPLC systems may be used. One of skill in the art would recognize that other methods may be used (e.g., UPLC). Samples may be subjected to plate or hemocytometer counts to assess microbial populations. One of skill in the art would recognize that other methods may be used (e.g., fluorescence microscopy, flow cytometry, and the like). Samples may also be assayed for levels of cellulose, hemicellulose, lignin, starch, and pectin using National Renewable Energy Laboratory procedures.

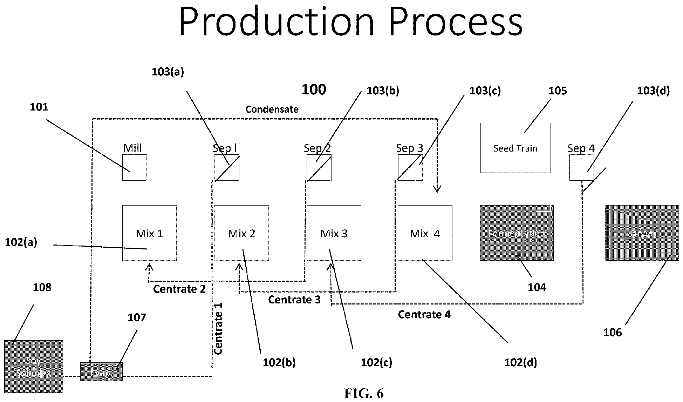

FIGs. 6-9 illustrate production processes 100, 100(a), 100(b), 100(c) that may be used to generate HQPC as disclosed herein. Referring to FIG. 6, for embodiments, plant-based material is first subject to milling device 101, and subsequently transferred to first mix tank 102(a), where it is mixed with one or more first solvents, which first solvents may contain acids, bases, enzymes, defoamer, and/or centrate from a downstream separation step (e.g., centrate 2, during continuous cycling). Enzymes include non-cellulose deconstructing enzymes (e.g., phytase, proteases and the like). The resulting mash is separated from the one or more first solvents by hydrodynamic force device (e.g., decanting centrifuge) 103(a) to produce a first centrate and a first cake. The first centrate may be transferred to evaporator 107 to produce soy solubles 108 (i.e., liquid protein concentrate). The first cake is transferred to a second mix tank 102(b), where the first cake is washed with one or more second solvents and/or a downstream centrate (e.g., centrate 3, for continuous cycling). The washed first cake is separated from the one or more second solvents by hydrodynamic force device 103(b) to produce a second centrate and a washed second cake. The second centrate may be transferred to first mix tank 102(a) during continuous cycling. The washed second cake is transferred to third mix tank 102(c), where the washed second cake is washed with one or more third solvents and/or a downstream centrate (e.g., centrate 3, for continuous cycling). The washed second cake is separated from the one or more third solvents by hydrodynamic force device 103(c) to produce a third centrate and a washed third cake. The third centrate may be transferred to second mix tank 102(b) during continuous cycling. The washed third cake is transferred to fourth mix tank 102(d), where the washed third cake is washed with one or more third solvents and/or a condensate from evaporator 107 to form a suspension, which suspension is transferred to one or more fermenters 104 for inoculation by seed train 105. Prior to incubation, the suspension may be heated and cooled in cook-tube/cooler tandem devices (not shown), where the residence time and temperature in said cook-tube may be modulated to change the characteristics of the final product (e.g., increase protein content). In embodiments, the residence time may be 0 to about 5 secs, about 5 secs to about 10 secs, about 10 secs to about 15 secs, about 20 secs to about 30 secs, or about 30 secs to 1 min. In a related aspect, the suspension is heated in the cook-tube at a temperature of at least about 95° C, about 105° C, about 110° C, about 120° C, about 130° C, or about 140° C, where the suspension is cooled to between about 30° to 32° C prior to incubation with seed train 105.

Continuing from FIG. 6, after incubation, the resulting fermented suspension is separated into a fourth centrate and final cake by application of hydrodynamic force device (e.g., decanting centrifuge/disc stack centrifuge) 103(d). The suspension may be heated in a broth tank (not shown) to at least about 60° C, for about 30 mins to about 2 hr, prior to application of hydrodynamic force. The resulting cake from 103(d) and/or the broth tank (not shown) is transferred to drier 106. Drying is carried out by heating drier 106 to about 150° C until the ultimate cake has a moisture content of less than about 7%.

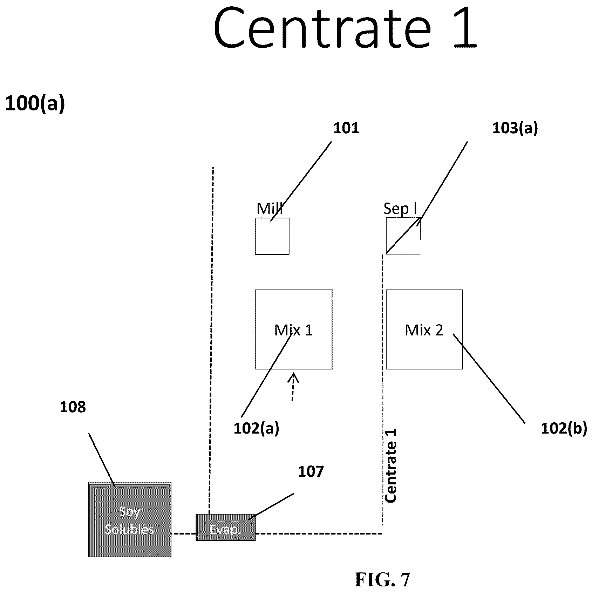

Referring to FIG. 7, the drawing provides a detail of the first steps of the production process 100(a) (centrate 1). Plant-based material is first subject to milling device 101, and subsequently transferred to first mix tank 102(a), where it is mixed with one or more first solvents. The resulting mash is separated from the one or more first solvents by hydrodynamic force device 103(a) to produce a first centrate and a first cake. The first centrate may be transferred to evaporator 107 to produce soy solubles 108.

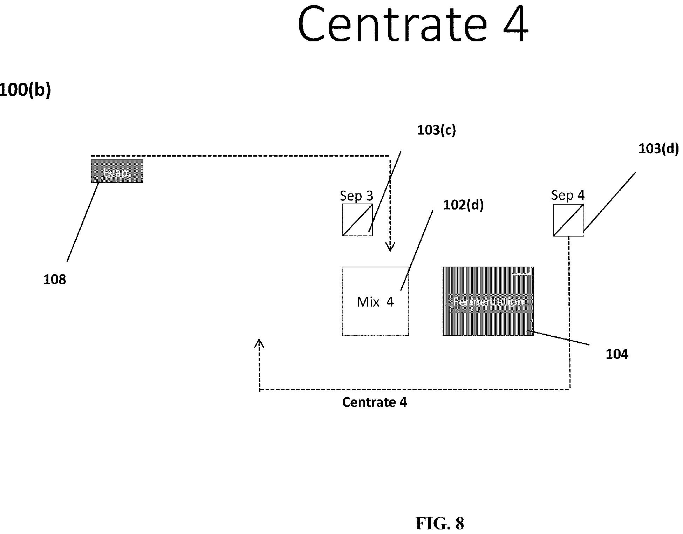

Referring to FIG. 8, the drawing provides production process detail for centrate 4 100(b). The washed third cake is transferred to fourth mix tank 102(d), where the washed third cake is washed with one or more third solvents and/or a condensate from evaporator 107 to form a suspension, which suspension is transferred to one or more fermenters 104 for inoculation. After incubation, the resulting fermented suspension is separated into a fourth centrate and final cake by application of hydrodynamic force device 103(d), where centrate 4 is transferred to an upstream mix tank (e.g., 102(c)).

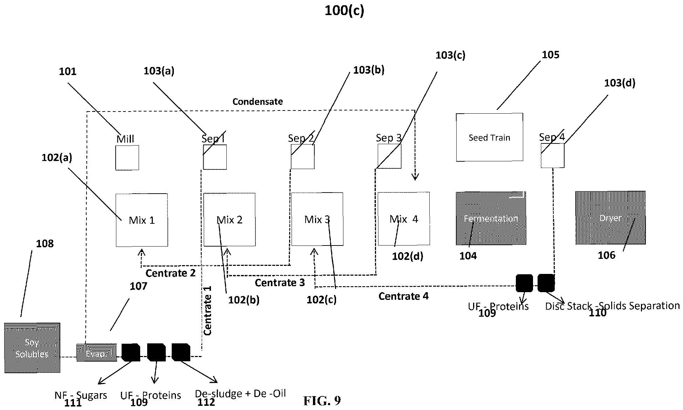

Referring to FIG. 9, production process 100(c) provides details relating to centrates 4 and 1, where centrate 4 is subject to disc stack centrifugation 109 and/or ultrafiltration 110 prior to transfer to mix tank 3 102(c), including that centrate 1 may be subject to de-sludge and de-oil, ultrafiltration 110 for protein separation, and/or nanofiltration 111 for sugar separation. In embodiments, such separation methods may be used to remove microbes used in the fermentation.

In embodiments, referring to FIGs. 6-9, production process 100, 100(a), 100(b), 100(c) may be batch or continuous, and contain at minimum one wash cycle (from mill 101 to dryer 106), and at least two centrates, where one centrate is further processed to concentrate soy solubles 108.

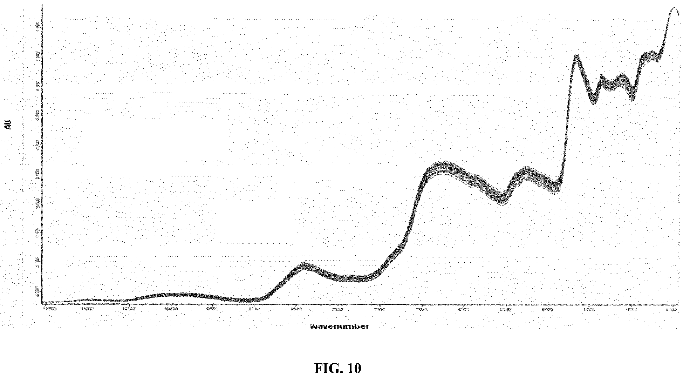

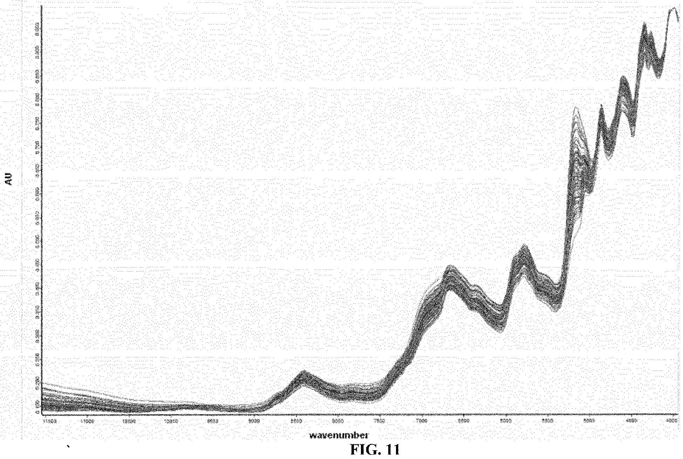

In embodiments, the feed stock prior to milling and the final product (ultimate dried cake) are analyzed by NIR spectroscopy. In one aspect, there should be a significant shift downward in the raw spectra between 4664 cm$^{-1}$ and 4836 cm$^{-1}$ for the final product (i.e., a "valley") relative to the feed stock (i.e., substantially "flat") (see FIGs. 10 and 11). In a related aspect, the shift downward should be at least about a 10% change, between about a 10% to about a 15% change, or between about a 15% change and about a 20% change or greater, calculated using the equation:

$$(V_2-V_1)|V_1|\times 100 = \text{Percent Change}$$

For example, as shown between FIGs. 10 and 11, the percent change in downward shift was about 16.7% for an SBM sample and a final ME-PRO® (HSPQ product) sample from which it was produced. While not being bound by theory, such a shift is indicative of increased protein content (see, e.g., Fan et al., PLoS ONE (2016) 11(9):e0163145. doi:10.1371/journal. pone.0163145). NIR spectrum data may be generated using the methods as disclosed in Fan et al. ((2016); herein incorporated by reference in its entirety), however, the skilled artisan would recognize that other suitable methods, including any associated hardware and software, are available.

Dietary Formulations

In exemplary embodiments, the HQPC recovered from the conversion culture that has undergone conversion is used in dietary formulations. In embodiments, the recovered HQPC may be the only protein source in the dietary formulation. Protein source percentages in dietary formulations are not meant to be limiting and may include 24 to 80% protein concentrate. In embodiments, HQPC will be more than about 50%, more than about 60%, or more than about 70% of the total dietary formulation protein source. Recovered HQPC may replace/supplement protein sources such as fish meal, soybean meal, wheat and corn flours and glutens and concentrates, and animal byproduct such as blood, poultry, meat substitutes, meat analogs, and feather meals. Dietary formulations using HQPC may also include supplements such as mineral and vitamin premixes to satisfy remaining nutrient requirements as appropriate.

In certain embodiments, performance of the HQPC, may be measured by comparing the growth, feed conversion, protein efficiency, DH, APD, PPD, and survival of animal on a high-quality protein concentrate dietary formulation to animals fed control dietary formulations, including aesthetics for foodstuff for human consumption. In embodiments, test formulations contain consistent protein, lipid, and energy contents. For example, when the animal is a fish, viscera (fat deposition) and organ (liver and spleen) characteristics, dress-out percentage, and fillet proximate analysis, as well as intestinal histology (enteritis) may be measured to assess dietary response. In embodiments, for juvenile shrimp formulations, in vitro DH, PPD, and ADP may be measured to assess dietary response. In embodiments, for piglets, gut infections characterized with diarrhea are the major cause of reduced growth performance and increased morbidity and mortality of post-weaned pigs, thus, reduction in diarrhea may be measured to assess dietary response.

As is understood, individual dietary formulations containing the recovered HQPC may be optimized for different kinds of animals. In embodiments, the animals include, but are not limited to, fin-fish, crustaceans (e.g., shrimp, crabs, prawns, and lobsters), domestic animals (e.g., dogs, cats, birds) and farm animals (e.g., cattle, pigs, and chickens). In a related aspect, the animals are fin-fish and crustaceans that are grown in commercial aquaculture. In another related aspect, the crustaceans include juvenile shrimp. Methods for optimization of dietary formulations are well-known and easily ascertainable by the skilled artisan without undue experimentation.

Complete grower diets may be formulated using HQPC in accordance with known nutrient requirements for various animal species. In embodiments, the formulation may be used for yellow perch (e.g., 42% protein, 8% lipid). In embodiments, the formulation may be used for rainbow trout (35% protein, 16% lipid). In embodiments, the formulation may be used for any one of the animals recited *supra.*

Basal mineral and vitamin premixes for plant-based diets may be used to ensure that micro-nutrient requirements will be met. Any supplements (as deemed necessary by analysis) may be evaluated by comparing to an identical formulation without supplementation; thus, the feeding trial may be done in a factorial design to account for supplementation effects. In embodiments, feeding trials may include a fish meal-based control diet and ESPC- and LSPC-based reference diets [traditional SPC (TSPC) is produced from solvent washing soy flake to remove soluble carbohydrate; texturized SPC (ESPC) is produced by extruding TSPC under moist, high temperature; and low-antigen SPC (LSPC) is produced from TSPC by altering the solvent wash and temperature during processing]. Pellets for feeding trials may be produced using a single screw extruder (e.g., BRABENDERPLASTI-CORDER EXTRUDER Model PL2000).

Feeding Trials

In embodiments, a replication of four experimental units per treatment (i.e., each experimental and control diet blend) may be used (e.g., about 60 to 120 days each). Trials may be carried out in 110-L circular tanks (20 fish/tank) connected in parallel to a closed-loop recirculation system driven by a centrifugal pump and consisting of a solids sump, and bioreactor, filters (100 μm bag, carbon and ultra-violet). Heat pumps may be used as required to maintain optimal temperatures for species-specific growth. Water quality (e.g., dissolved oxygen, pH, temperature, ammonia and nitrite) may be monitored in all systems.

In embodiments, experimental diets may be delivered according to fish size and split into two to five daily feedings. Growth performance may be determined by total mass measurements taken at one to four weeks (depending upon fish size and trial duration); rations may be adjusted in accordance with gains to allow satiation feeding and to reduce waste streams. Consumption may be assessed biweekly from collections of uneaten feed from individual tanks. Uneaten feed may be dried to a constant temperature, cooled, and weighed to estimate feed conversion efficiency. Protein and energy digestibilities may be determined from fecal material manually stripped during the midpoint of each experiment or via necropsy from the lower intestinal tract at the conclusion of a feeding trial. Survival, weight gain, growth rate, health indices, feed conversion, protein and energy digestibilities, and protein efficiency may be compared among treatment groups. Proximate analysis of necropsied fishes may be carried out to compare composition of fillets among dietary treatments. Analysis of amino and fatty acids may be done as needed for fillet constituents, according to the feeding trial objective. Feeding trial responses of dietary treatments may be compared to a control (e.g., fish meal) diet response to ascertain whether performance of HQPC diets meet or exceed control responses.

For shrimp, trials may be conducted in either 900-gallon tanks or semisquare 190 L tanks each equipped with a recirculating drain which withdraws water from the subsurface and a sludge drain which is affixed to the lowest point in the bottom at the center of the tank. The RAS testing system may consist of Cornell-style dual drain tanks, solids settling tanks, mechanical drum filtration, moving bed bioreactor (MBBR), UV sterilization, chilling, and oxygen injection. Water quality may be monitored on a daily basis to ensure all parameters are within acceptable ranges (see, e.g., White et al., Aqua Mar Bio Eco (2020) JAMBE:105).

Further, shrimp may be analyzed to determine direct effects of HQPC such as providing significant amounts of biologically-active factors that can increase gut micro-biota, reduce intestinal-inflammation and boost metabolic processes for improved animal health, which may depend, inter alia, on dry matter conversion of feed to weight, where the determination of digestibility plays a key role. In a related aspect, in vitro protein digestion with standardized digestive enzymes recovered from shrimp may be used, including using the data from such analysis to determine DH, PPD and APD, as well as overall survival.

Statistical analyses of diets and feeding trial responses may be completed with an *a priori a* = 0.05. Analysis of performance parameters among treatments may be performed with appropriate analysis of variance or covariance (Proc Mixed) and post hoc multiple comparisons, as needed. Analysis of animal performance and tissue responses may be assessed by nonlinear models.

In embodiments, the present disclosure proposes to convert fibers and other carbohydrates in plant-based materials into additional protein using, for example, a GRAS-status microbe. A microbial exopolysaccharide (e.g., glucans) may also be produced that may facilitate extruded feed pellet formation, negating the need for binders. This microbial gum may also provide immunostimulant activity to activate innate defense mechanisms that protect animals from common pathogens resulting from stressors. Immunoprophylactic substances, such as β-glucans, bacterial products, and plant constituents, are increasingly used in commercial feeds to reduce economic losses due to infectious diseases and minimize antibiotic use. The microbes of the present disclosure also produce extracellular peptidases, which should increase protein digestibility and absorption during metabolism, providing higher feed efficiency and yields. As disclosed herein, this microbial incubation process provides a valuable, sustainable plant-protein based feed that is less expensive per unit of protein than SBM, SPC, and animal feeds. In embodiments, the centrate components can be subjected further afterwards to an evaporation step that can concentrate soluble coproducts, such as sugars, glycerol, proteins, peptides and amino acids, into a material called syrup or condensed plant based solubles (PBS).

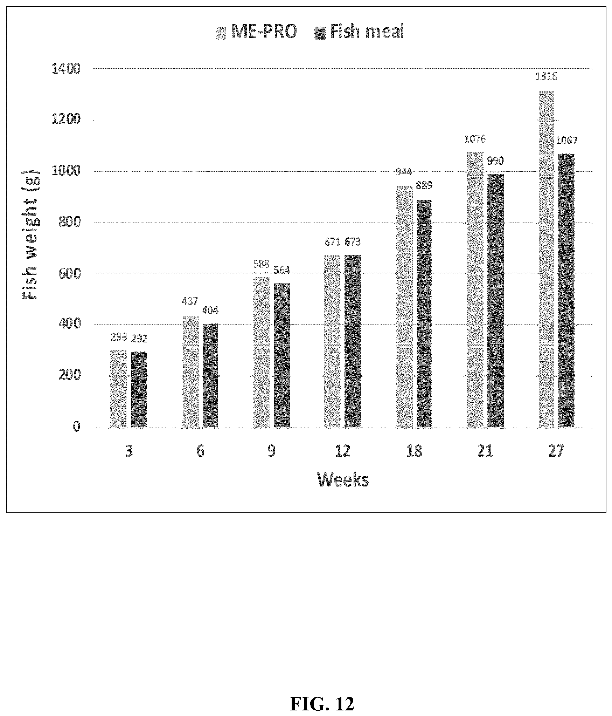

As disclosed, the instant microbes may metabolize the individual carbohydrates in plant-based materials, producing both cell mass (protein), a microbial gum (e.g., pullulan), enzyme production, microbial metabolite production, and probiotics. Various strains of these microbes also enhance fiber deconstruction. The microbes of the present invention may also convert plant-based material proteins into more digestible peptides and amino acids (electro here FIG 12). In embodiments, the following actions may be performed: 1) determining the efficiency of using select microbes of the present disclosure to convert plant based materials, yielding a high quality protein concentrate (HQPC) with a protein concentration of at least between about 65% to about 70% or greater, and 2) assessing the effectiveness of HQPC in replacing animal-based protein. In embodiments, optimizing process/conversion conditions may be carried out to improve the performance and robustness of the microbes, test the resultant grower feeds for a range of commercially important animals, validate process costs and energy requirements for commercialization. In embodiments, the HQPC of the present disclosure may be able to replace at least 50% of animal proteins, while providing increased growth rates and conversion efficiencies. For example, production costs should be less than commercial soy protein concentrate (SPC) and substantially less than fish meal.

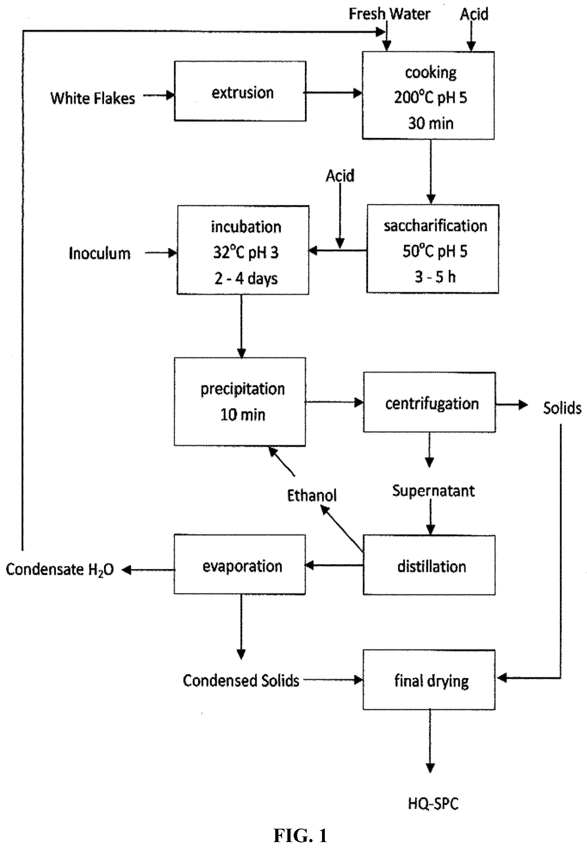

Figures 1, 2 and 6-9 show various approaches of the present disclosure in the treatment of a plant based product, converting sugars into cell mass (protein) and gum, recovering HQPC and generating aqua feeds, and testing the resulting aqua feeds in fish feeding trials.

While not required for all processes disclosed herein, cellulose-deconstructing enzymes/pullulanases may be evaluated to generate sugars, which microbes of the present disclosure may convert to protein, exopolysaccharides and gum. In embodiments, sequential omission of these enzymes and evaluation of co-culturing with cellulolytic microbes may be used. Ethanol may be evaluated to wash the various cakes, precipitate the gum and improve recovery of protein solubles that may be suspended in various centrates through processing to generate HQPC. After drying, the HQPC may be incorporated into practical diet formulations. In embodiments, test grower diets may be formulated (with mineral and vitamin premixes) and comparisons to an animal protein control and commercial plant-based protein concentrate/isolate based diets in feeding trials with commercially important animals may be performed. Performance (e.g., growth, feed conversion, protein efficiency, survival), viscera characteristics, gut affects, and intestinal histology may be examined to assess responses.

In other embodiments, optimizing the HQPC production process by determining optimum conversion conditions while minimizing process inputs, improving the performance and robustness of the microbe, testing the resultant grower feeds for a range of commercially important animals, and validating/updating process costs and energy requirements may be conducted.

In the past few years, a handful of facilities have installed a dry mill capability that removes corn hulls and germ prior to the ethanol production process. This dry fractionation process yields a DDGS with up to 42% protein (hereafter referred to as dryfrac DDGS). In some embodiments, low oil DDGS may be used as a substrate for conversion, where such low oil DDGS has a higher protein level than conventional DDGS. In a related aspect, low oil DDGS increase growth rates of *A. pullulans* compared to conventional DDGS.

Several groups are evaluating partial replacement of animal based protein with plant derived proteins. However, the lower protein content, inadequate amino acid balance, and presence of anti-nutritional factors have limited the replacement levels to 20-40%. For example, preliminary growth trials indicate that no current DDGS or SPC-based diets provide performance similar to fish-meal control diets. Several deficiencies have been identified among commercially produced DDGS and SPCs, principally in protein and amino acid composition, which impart variability in growth performance. However, plant-based protein containing diets as disclosed herein containing nutritional supplements (formulated to meet or exceed all requirements) have provided growth results that are similar to or exceed animal protein-based controls. Thus, the processes as disclosed herein and products developed therefrom provide a higher quality HQPC (relative to nutritional requirements) and support growth performance equivalent to or better than diets containing animal-based protein, including those containing various SPC/SPI.

In embodiments, fish that can be fed the fish feed composition of the present disclosure include, but are not limited to, Siberian sturgeon, Sterlet sturgeon, Starry sturgeon, White sturgeon, Arapaima, Japanese eel, American eel, Short-finned eel, Long-finned eel, European eel, Chanos chanos, Milkfish, Bluegill sunfish, Green sunfish, White crappie, Black crappie, Asp, Catla, Goldfish, Crucian carp, Mud carp, Mrigal carp, Grass carp, Common carp, Silver carp, Bighead carp, Orangefin labeo, Roho labeo, Hoven's carp, Wuchang bream, Black carp, Golden shiner, Nilem carp, White amur bream, Thai silver barb, Java, Roach, Tench, Pond loach, Bocachico, Dorada, Cachama, Cachama Blanca, Paco, Black bullhead, Channel catfish, Bagrid catfish, Blue catfish, Wels catfish, Pangasius (Swai, Tra, Basa) catfish, Striped catfish, Mudfish, Philippine catfish, Hong Kong catfish, North African catfish, Bighead catfish, Sampa, South American catfish, Atipa, Northern pike, Ayu sweetfish, Vendace, Whitefish, Pink salmon, Chum salmon, Coho salmon, Masu salmon, Rainbow trout, Sockeye salmon, Chinook salmon, Atlantic salmon, Sea trout, Arctic char, Brook trout, Lake trout, Atlantic cod, Pejerrey, Lai, Common snook, Barramundi/Asian sea bass, Nile perch, Murray cod, Golden perch, Striped bass, White bass, European seabass, Hong Kong grouper, Areolate grouper, Greasy grouper, Spotted coralgrouper, Silver perch, White perch, Jade perch, Largemouth bass, Smallmouth bass, European perch, Zander (Pike-perch), Yellow Perch, Sauger, Walleye, Bluefish, Greater amberjack, Japanese amberjack, Snubnose pompano, Florida pompano, Palometa pompano, Japanese jack mackerel, Cobia, Mangrove red snapper, Yellowtail snapper, Dark seabream, White seabream, Crimson seabream, Red seabream, Red porgy, Goldlined seabream, Gilthead seabream, Red drum, Green terror, Blackbelt cichlid, Jaguar guapote, Mexican mojarra, Pearlspot, Three spotted tilapia, Blue tilapia, Longfin tilapia, Mozambique tilapia, Nile tilapia, Tilapia, Wami tilapia, Blackchin tilapia, Redbreast tilapia, Redbelly tilapia, Golden grey mullet, Largescale mullet, Gold-spot mullet, Thinlip grey mullet, Leaping mullet, Tade mullet, Flathead grey mullet, White mullet, Lebranche mullet, Pacific fat sleeper, Marble goby, White-spotted spinefoot, Goldlined spinefoot, Marbled spinefoot, Southern bluefin tuna, Northern bluefin tuna, Climbing perch, Snakeskin gourami, Kissing gourami, Giant gourami, Snakehead, Indonesian snakehead, Spotted snakehead, Striped snakehead, Turbot, Bastard halibut (Japanese flounder), Summer Flounder, Southern flounder, Winter flounder, Atlantic Halibut, Greenback flounder, Common sole, and combinations thereof

In embodiments, crustaceans that can be fed the feed composition of the present disclosure include, but are not limited to, Kadal shrimp, endeavour shrimp, greasyback shrimp, speckled shrimp, northern brown shrimp, fleshy prawn, brown tiger prawn, Indian white prawn, Kuruma prawn, caramote prawn, banana prawn, giant tiger prawn, southern pink shrimp, Sao Paulo shrimp, redtail prawn, southern white shrimp, Green tiger prawn, Northern white shrimp, Blue shrimp, Southern brown shrimp, Whiteleg shrimp, Atlantic seabob, Akiami paste shrimp, Monsoon river prawn, giant river prawn, common prawn, American lobster, European lobster, noble crayfish, Danube crayfish, signal crayfish, red swamp crawfish, yabby crayfish, red claw crayfish, marron crayfish, longlegged spiny lobster, gazami crab, Indo-Pacific swamp crab, Chinese river crab, and combinations thereof.

In embodiments, farm animals that can be fed the feed composition of the present disclosure include, but are not limited to, cattle, sheep, goats, deer, horses, chickens, pigs, rabbits, ducks, alpaca, emus, turkeys, bison, and camels.

In embodiments, domestic animals that can be fed the feed compositions of the present disclosure include, but are not limited to, dogs, cats, parrots, gold fish, turtles, parakeets, hamsters, lab rats, guinea pigs, and lab mice.

It will be appreciated by the skilled person that the feed composition of the present disclosure may be used as a convenient carrier for pharmaceutically active substances including, but not limited to, antibiotics, chemotherapeutics, anti-inflammatories, NSAIDs, antimicrobial agents and immunologically active substances including vaccines against bacterial or viral infections, and any combination thereof.

The feed composition according to the present disclosure may be provided as a liquid, pourable emulsion, or in the form of a paste, or in a dry form, for example as a granulate, a powder, or as flakes. When the feed composition is provided as an emulsion, a lipid-in-water emulsion, it may be in a relatively concentrated form. Such a concentrated emulsion form may also be referred to as a pre-emulsion as it may be diluted in one or more steps in an aqueous medium to provide the final enrichment medium for the organisms.

In embodiments, cellulosic-containing starting material for the microbial-based process as disclosed is corn. Corn is about two-thirds starch, which is converted during a fermentation and distilling process into ethanol and carbon dioxide. The remaining nutrients or fermentation products may result in condensed distiller's solubles or distiller's grains such as DDGS, which can be used in feed products. In general, the process involves an initial preparation step of dry milling or grinding of the corn. The processed corn is then subjected to hydrolysis and enzymes added to break down the principal starch component in a saccharification step. The following step of fermentation is allowed to proceed upon addition of a microorganism (e.g., yeast) provided in accordance with an embodiment of the disclosure to produce gaseous products such as carbon dioxide. The fermentation is conducted for the production of ethanol which can be distilled from the fermentation broth. The remainder of the fermentation medium can be then dried to produce fermentation products including DDGS. This step usually includes a solid/liquid separation process by centrifugation wherein a solid phase component can be collected. Other methods including filtration and spray dry techniques can be employed to effect such separation. The liquid phase components can be subjected further afterwards to an evaporation step that can concentrate soluble coproducts, such as sugars, glycerol, proteins, peptides and amino acids, into a material called syrup or condensed corn solubles (CCS). The CCS can then be recombined with the solid phase component to be dried as incubation products (DDGS). It shall be understood that the subject compositions can be applied to new or already existing ethanol plants based on dry milling to provide an integrated ethanol production process that also generates fermentation products with increased value.

In embodiments, incubation products produced according to the present disclosure have a higher commercial value than the conventional fermentation products. For example, the incubation products may include enhanced dried solids with improved amino acid and micronutrient content. A "golden colored" product can be thus provided which generally indicates higher amino acid digestibility compared to darker colored HQSP. For example, a light-colored HQSP may be produced with an increased lysine concentration in accordance with embodiments herein compared to relatively darker colored products with generally less nutritional value. The color of the products may be an important factor or indicator in assessing the quality and nutrient digestibility of the fermentation products or HQSP. Color is used as an indicator of exposure to excess heat during drying causing caramelization and Millard reactions of the free amino groups and sugars, reducing the quality of some amino acids.

Another aspect of the present invention is directed towards complete feed compositions with an enhanced concentration of nutrients which includes microorganisms characterized by an enhanced concentration of nutrients such as, but not limited to, fats, fatty acids, lipids such as phospholipid, vitamins, essential amino acids, peptides, proteins, carbohydrates, sterols, enzymes, and trace minerals such as, iron, copper, zinc, manganese, cobalt, iodine, selenium, molybdenum, nickel, fluorine, vanadium, tin, silicon, and combinations thereof. In one aspect, the process makes available a greater amount of phosphorous to the animal in a manner that does not lead to substantial contamination of waste water.

The incubation products obtained after the incubation process are typically of higher commercial value. In embodiments, the incubation products contain microorganisms that have enhanced nutrient content than those products deficient in the microorganisms. The microorganisms may be present in an incubation system, the incubation broth and/or incubation biomass. The incubation broth and/or biomass may be dried (e.g., spray-dried), to produce the incubation products with an enhanced content of the nutritional contents.

For example, the spent, dried solids recovered following the incubation process are enhanced in accordance with the disclosure. These incubation products are generally non-toxic, biodegradable, readily available, inexpensive, and rich in nutrients. The choice of microorganism and the incubation conditions are important to produce a low toxicity or non-toxic incubation product for use as a feed or nutritional supplement, including selection of a microorganism that does not produce metabolites in concentrations that would otherwise negatively affect the process. While glucose is the major sugar produced from the hydrolysis of the starch from grains, it is not the only sugar produced in carbohydrates generally. Unlike the SPC or DDG produced from the traditional dry mill ethanol production process, which contains a large amount of non-starch carbohydrates (e.g., as much as 35% of cellulose and arabinoxylans-measured as neutral detergent fiber, by dry weight), the subject nutrient enriched incubation products produced by enzymatic hydrolysis of the non-starch carbohydrates (via metabolism by the microorganism) are more palatable and digestible to the non-ruminant.

The nutrient enriched incubation product of this disclosure may have a nutrient content of from at least about 1% to about 95% by weight. The nutrient content is preferably in the range of at least about 10%-20%, 20%-30%, 30%-40%, 40%-50%, 50%-60%, 60%-70%, and 70%-80% by weight. The available nutrient content may depend upon the animal to which it is fed and the context of the remainder of the diet, and stage in the animal life cycle. For instance, beef cattle require less histidine than lactating cows. Selection of suitable nutrient content for feeding animals is well known to those skilled in the art.

The incubation products may be prepared as a spray-dried biomass product. Optionally, the biomass may be separated by known methods, such as centrifugation, filtration, separation, decanting, a combination of separation and decanting, ultrafiltration or microfiltration. The biomass incubation products may be further treated to facilitate rumen bypass. In embodiments, the biomass product may be separated from the incubation medium, spray-dried, and optionally treated to modulate rumen bypass, and added to feed as a nutritional source. In addition to producing nutritionally enriched incubation products in an incubation process containing microorganisms, the nutritionally enriched incubation products may also be produced in transgenic plant systems. Methods for producing transgenic plant systems are known in the art. Alternatively, where the microorganism host excretes the nutritional contents, the nutritionally-enriched broth may be separated from the biomass produced by the incubation and the clarified broth may be used as an animal feed ingredient, e.g., either in liquid form or in spray dried form.

The incubation products obtained after the incubation process using microorganisms may be used as an animal feed or as food supplement for humans. The incubation product includes at least one ingredient that has an enhanced nutritional content that is derived from a non-animal source (e.g., a bacteria, yeast, and/or plant). In particular, the incubation products are rich in at least one or more of fats, fatty acids, lipids such as phospholipid, vitamins, essential amino acids, peptides, proteins, carbohydrates, sterols, enzymes, and trace minerals such as, iron, copper, zinc, manganese, cobalt, iodine, selenium, molybdenum, nickel, fluorine, vanadium, tin and silicon. In embodiments, the peptides contain at least one essential amino acid. In other embodiments, the essential amino acids are encapsulated inside a subject modified microorganism used in an incubation reaction. In embodiments, the essential amino acids are contained in heterologous polypeptides expressed by the microorganism. Where desired, the heterologous polypeptides are expressed and stored in the inclusion bodies in a suitable microorganism (e.g., fungi).

In embodiments, the incubation products have a high nutritional content. As a result, a higher percentage of the incubation products may be used in a complete animal feed. In embodiments, the feed composition comprises at least about 15% of incubation product by weight. In a complete feed, or diet, this material will be fed with other materials. Depending upon the nutritional content of the other materials, and/or the nutritional requirements of the animal to which the feed is provided, the modified incubation products may range from 15% of the feed to 100% of the feed. In embodiments, the subject incubation products may provide lower percentage blending due to high nutrient content. In other embodiments, the subject incubation products may provide very high fraction feeding, e.g. over 75%. In suitable embodiments, the feed composition comprises at least about 20%, at least about 25%, at least about 30%, at least about 35%, at least about 40%, at least about 45%, at least about 50%, at least about 60%, at least about 70%, or at least about 75% of the subject incubation products. Commonly, the feed composition comprises at least about 20% of incubation product by weight. More commonly, the feed composition comprises at least about 15-25%, 25-20%, 20-25%, 30%-40%, 40%-50%, 50%-60%, or 60%-70% by weight of incubation product. Where desired, the subject incubation products may be used as a sole source of feed.

In embodiments, a dietary formulation as disclosed herein may have enhanced amino acid content with regard to one or more essential amino acids for a variety of purposes, e.g., for weight increase and overall improvement of the animal's health. The formulations may have an enhanced amino acid content because of the presence of free amino acids and/or the presence of proteins or peptides including an essential amino acid, in the incubation products. Essential amino acids may include histidine, lysine, methionine, phenylalanine, threonine, taurine, isoleucine, and/or tryptophan, which may be present in the formulation as a free amino acid or as part of a protein or peptide that is rich in the selected amino acid. At least one essential amino acid-rich peptide or protein may have at least 1% essential amino acid residues per total amino acid residues in the peptide or protein, at least 5% essential amino acid residues per total amino acid residues in the peptide or protein, or at least 10% essential amino acid residues per total amino acid residues in the protein. By feeding a diet balanced in nutrients to animals, maximum use is made of the nutritional content, requiring less feed to achieve comparable rates of growth, milk production, or a reduction in the nutrients present in the excreta reducing bioburden of the wastes (e.g., reduces phosphorous in waste streams).

A formulation as disclosed herein may contain an enhanced content of an essential amino acid, may have an essential amino acid content (including free essential amino acid and essential amino acid present in a protein or peptide) of at least 2.0 wt % relative to the weight of the crude protein and total amino acid content, and more suitably at least 5.0 wt % relative to the weight of the crude protein and total amino acid content. A feed formulation as disclosed herein includes other nutrients derived from microorganisms including but not limited to, fats, fatty acids, lipids such as phospholipid, vitamins, carbohydrates, sterols, enzymes, and trace minerals.

A feed formulation as disclosed herein may include complete feed form composition, concentrate form composition, blender form composition, and base form composition. If the formulation is in the form of a complete feed, the percent nutrient level, where the nutrients are obtained from the microorganism in an incubation product, which may be about 10 to about 25 percent, more suitably about 14 to about 24 percent; whereas, if the formualtion is in the form of a concentrate, the nutrient level may be about 30 to about 50 percent, more suitably about 32 to about 48 percent. If the formulation is in the form of a blender, the nutrient level in the composition may be about 20 to about 30 percent, more suitably about 24 to about 26 percent; and if the formulation is in the form of a base mix, the nutrient level in the formulation may be about 55 to about 65 percent. Unless otherwise stated herein, percentages are stated on a weight percent basis. If the HQPC is high in a single nutrient, e.g., Lys, it will be used as a supplement at a low rate; if it is balanced in amino acids and Vitamins, e.g., vitamin A and E, it will be a more complete feed and will be fed at a higher rate and supplemented with a low protein, low nutrient feed stock, like corn stover.

The feed formulation as disclosed herein may include a peptide or a crude protein fraction present in an incubation product having an essential amino acid content of at least about 2%. In embodiments, a peptide or crude protein fraction may have an essential amino acid content of at least about 3%, at least about 5%, at least about 10%, at least about 15%, at least about 20%, at least about 30%, at least about 40%, and in embodiments, at least about 50%. In embodiments, the peptide may be 100% essential amino acids. For example, a fish meal formulation may include a peptide or crude protein fraction present in an incubation product having an essential amino acid content of up to about 10%. More commonly, the fish meal formulation may include a peptide or a crude protein fraction present in an incubation product having an essential amino acid content of about 2-10%, 3.0-8.0%, or 4.0-6.0%.

The formulation as disclosed herein may include a peptide or a crude protein fraction present in a incubation product having a lysine content of at least about 2%. In embodiments, the peptide or crude protein fraction may have a lysine content of at least about 3%, at least about 5%, at least about 10%, at least about 15%, at least about 20%, at least about 30%, at least about 40%, and in embodiments, at least about 50%. For example, a fish meal formulation may include the peptide or crude protein fraction having a lysine content of up to about 10%. Where desired, the fish meal formulation may include the peptide or a crude protein fraction having a lysine content of about 2-10%, 3.0-8.0%, or 4.0-6.0%.

The formulation as disclosed herein may include nutrients in the incubation product from about 1 g/Kg dry solids to 900 g/Kg dry solids. For example, nutrients in a fish meal formulation may be present to at least about 2 g/Kg dry solids, 5 g/Kg dry solids, 10 g/Kg dry solids, 50 g/Kg dry solids, 100 g/Kg dry solids, 200 g/Kg dry solids, and about 300 g/Kg dry solids. In embodiments, the nutrients may be present to at least about 400 g/Kg dry solids, at least about 500 g/Kg dry solids, at least about 600 g/Kg dry solids, at least about 700 g/Kg dry solids, at least about 800 g/Kg dry solids and/or at least about 900 g/Kg dry solids.

The formulation as disclosed herein may include an essential amino acid or a peptide containing at least one essential amino acid present in an incubation product having a content of about 1 g/Kg dry solids to 900 g/Kg dry solids. For example, the essential amino acid or a peptide containing at least one essential amino acid in a fish meal composition may be present to at least about 2 g/Kg dry solids, 5 g/Kg dry solids, 10 g/Kg dry solids, 50 g/Kg dry solids, 100 g/Kg dry solids, 200 g/Kg dry solids, and about 300 g/Kg dry solids. In embodiments, the essential amino acid or a peptide containing at least one essential amino acid may be present to at least about 400 g/Kg dry solids, at least about 500 g/Kg dry solids, at least about 600 g/Kg dry solids, at least about 700 g/Kg dry solids, at least about 800 g/Kg dry solids and/or at least about 900 g/Kg dry solids.

The formulation as disclosed herein may contain a nutrient enriched incubation product in the form of a biomass formed during incubation and at least one additional nutrient component. In another example, the formulation contains a nutrient enriched incubation product that is dissolved and suspended from an incubation broth formed during incubation and at least one additional nutrient component. In a further embodiment, the formulation has a crude protein fraction that includes at least one essential amino acid-rich protein. The formulation may be prepared so as to deliver an improved balance of essential amino acids.

For other formulations, the complete formulation may contain HQPC and one or more ingredients such as wheat middlings ("wheat mids"), corn, soybean meal, corn gluten meal, distiller's grains or distiller's grains with solubles, salt, macro-minerals, trace minerals and vitamins, generated with or without fermentation. Other potential ingredients may commonly include, but not be limited to sunflower meal, malt sprouts and soybean hulls. The blender formulation may contain wheat middlings, corn gluten meal, distiller's grains or distiller's grains with solubles, salt, macro-minerals, trace minerals and vitamins. Alternative ingredients would commonly include, but not be limited to, corn, soybean meal, sunflower meal, cottonseed meal, malt sprouts and soybean hulls. The base form formulation may contain wheat middlings, corn gluten meal, and distiller's grains or distiller's grains with solubles. Alternative ingredients would commonly include, but are not limited to, soybean meal, sunflower meal, malt sprouts, macro-minerals, trace minerals and vitamins.

Highly unsaturated fatty acids (HUFAs) in microorganisms, when exposed to oxidizing conditions may be converted to less desirable unsaturated fatty acids or to saturated fatty acids. However, saturation of omega-3 HUFAs may be reduced or prevented by the introduction of synthetic antioxidants or naturally-occurring antioxidants, such as beta-carotene, vitamin E and vitamin C, into the feed. Synthetic antioxidants, such as BHT, BHA, TBHQ or ethoxyquin, or natural antioxidants such as tocopherols, may be incorporated into the food or feed products by adding them to the products, or they may be incorporated by in situ production in a suitable organism. The amount of antioxidants incorporated in this manner depends, for example, on subsequent use requirements, such as product formulation, packaging methods, and desired shelf life.

Incubation products of the present disclosure may also be utilized as a nutritional supplement for human consumption where the process begins with human grade input materials, and human food quality standards are observed throughout the process. Incubation product or the formulation as disclosed herein is high in nutritional content. Nutrients such as, protein and fiber are associated with healthy diets. Recipes may be developed to utilize incubation product or the complete feed of the disclosure in foods such as cereal, crackers, pies, cookies, cakes, pizza crust, summer sausage, meat balls, shakes, and in any forms of edible food. Another choice may be to develop the incubation product into snacks or a snack bar, similar to a granola bar that could be easily eaten, convenient to distribute. A snack bar may include protein, fiber, germ, vitamins, minerals, from the grain, as well as nutraceuticals such as glucosamine, HUFAs, or cofactors, such as Vitamin Q-10.

Formulations comprising the subject incubation products may be further supplemented with flavors. The choice of a particular flavor will depend on the animal to which the feed is provided. The flavors and aromas, both natural and artificial, may be used in making feeds more acceptable and palatable. These supplementations may blend well with all ingredients and may be available as a liquid or dry product form. Suitable flavors, attractants, and aromas to be supplemented in the animal feeds include but not limited to fish pheromones, fenugreek, banana, cherry, rosemary, cumin, carrot, peppermint, oregano, vanilla, anise, plus rum, maple, caramel, citrus oils, ethyl butyrate, menthol, apple, cinnamon, any natural or artificial combinations thereof. The flavors and aromas may be interchanged between different animals. Similarly, a variety of fruit flavors, artificial or natural may be added to food supplements comprising the subject incubation products for human consumption.

In embodiments, the HQPC may be part of a kit to generate various formulations, including the HQPC, a label, at least one container and instruction on generating formulations depending on the animal. Further, such instructions may be available through a weblink.

The shelf-life of the incubation product or the complete feed of the present disclosure may typically be longer than the shelf life of an incubation product that is deficient in the microorganism. The shelf-life may depend on factors such as, the moisture content of the product, how much air can flow through the feed mass, the environmental conditions and the use of preservatives. A preservative may be added to the complete feed to increase the shelf life to weeks and months. Other methods to increase shelf life include management similar to silage management such as mixing with other feeds and packing, covering with plastic or bagging. Cool conditions, preservatives and excluding air from the feed mass all extend shelf life of wet coproducts. The complete feed can be stored in bunkers or silo bags. Drying the wet incubation product or complete feed may also increase the product's shelf life and improve consistency and quality.

The complete feed of the present disclosure may be stored for long periods of time. The shelf life may be extended by ensiling, adding preservatives such as organic acids, or blending with other feeds such as soy hulls. Commodity bins or bulk storage sheds may be used for storing the complete feeds. In a related aspect, the HQPC as disclosed herein may have a shelf life of at least 2 years.

The following examples are illustrative and are not intended to limit the scope of the disclosed subject matter.

## EXAMPLES

Example 1. High Quality Protein Concentrate (HQPC) (precipitation method).

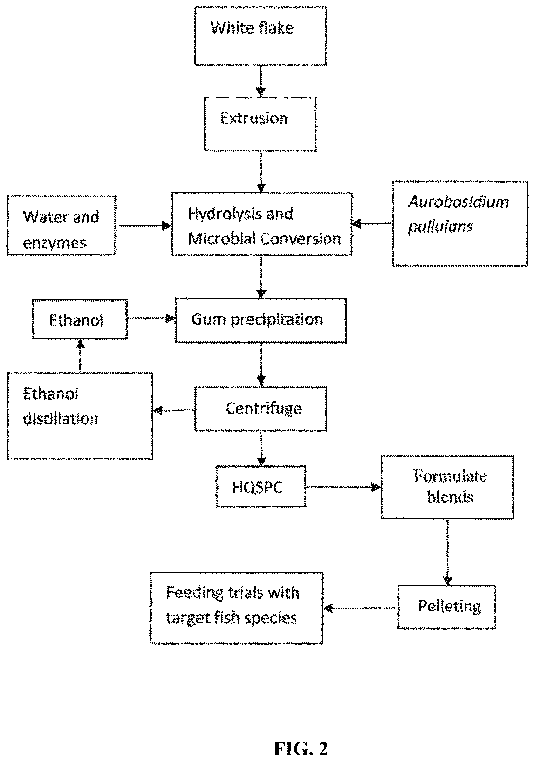

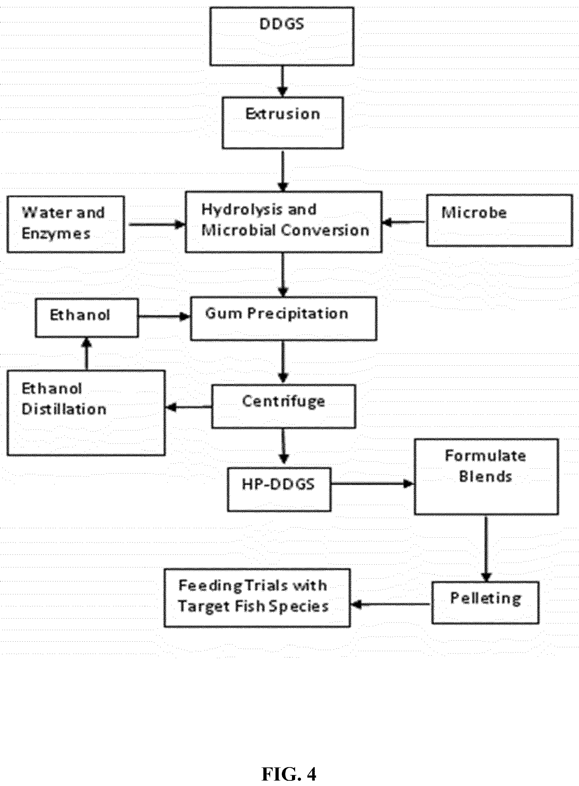

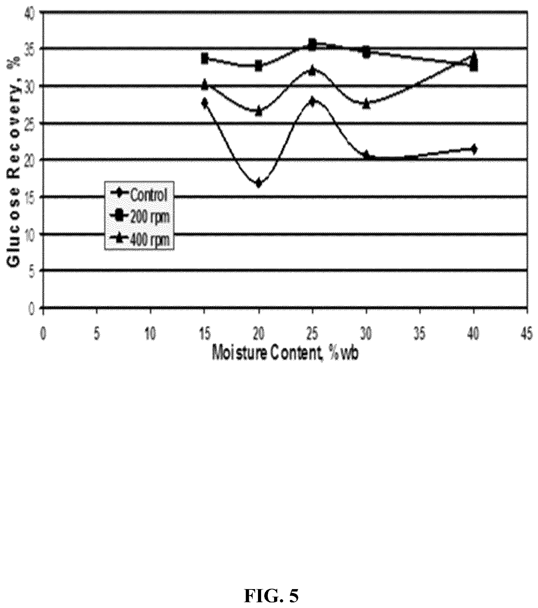

Figure 1 shows an approach to pre-treating white flakes, converting sugars into cell mass (proteinaceous material) and gum (e.g., exopolysaccharides), recovering HQSPC and generating aquafeeds (FIGs. 2 and 4), and testing resulting aquafeeds in fish feeding trials for a process. White flakes were first subject to extrusion pretreatment (BRABENDER PLASTI-CORDER SINGLE SCREW EXTRUDER Model PL2000, Hackensack, NJ) at 15% moisture content, 50° C, and 75 rpm to disrupt the structure and allow increased intrusion of hydrolytic enzymes during subsequent saccharification. These conditions provided a shearing effect against the rigged channels on both sides of the barrel, and it had been observed previously that this resulted in 50-70% greater sugar release following enzymatic hydrolysis. Extruded white flakes were then ground through a 3 mm hammermill screen, blended with water to achieve a 10% solid loading rate, and adjusted to pH 5. After heating to pasteurize or sterilize the mash, the mash was cooled to about 50° C and cellulose and oligosaccharide-deconstructing enzymes (15 ml total/kg of white flake) were added to hydrolyze the polymers into simple sugars (4-24h hydrolysis). Specific dosages included were 6% CELLIC CTEK (per gm glucan), 0.3% CELLIC HTEK (per gm total solids), 0.015% NOVOZYME 960 (per gm solids). The resulting mash was then cooled to 30° C, pH adjusted to 3-5, inoculated with *A. pullulans* (1% v/v), and incubated for 4-5 days at 50 to 200 rpm mixing and an aeration rate of 0.5 L/L/min to convert sugars into protein and gum. During incubation, samples were periodically removed and analyzed for sugars, cell counts and gum production. Following incubation, the pH was increased to 6.5, and ethanol (0.6 L/L broth) was added to precipitate the gum. The protein, pullulan and microbial mass (HQSPC) were recovered by centrifugation and dried, while the supernatant was distilled to recover ethanol, and the residual liquid chemically assayed for future recycling at the start of the process. FIG. 5 shows glucose recovery using ppt method.

HQSPC using Soy White Flake and Microbial Conversion with *A. Pullulans,* pilot scale system for the production of HQSPC.

The system contained a 675 L bioreactor, a variable speed progressive cavity pump, a continuous flow centrifuge, and a 1 x 4 meter drying table. Inoculum for use in the 675L bioreactor was prepared in two, 5L NEW BRUNSWICK BIOFLO 3 BIOREACTORS. For each trial 8-10L quantities of inoculum was prepared by growing *A. pullulan* as described for 2-3 days. This material was used to inoculate larger quantities of extruded and saccharified white flake prepared in the 675L bioreactor. Following incubation, ethanol was added, the mash was centrifuged to recover the wet solids which were then dried and used in fish feeding trials. By monitoring performance of the conversion process, the yield and composition of the HQSPC, several parameters were observed that significantly affected solids recovery. In the large scale trials, the parameters as shown in Table 1 were varied.

**Table 1. Pre-Pilot scale trail variables and key performance parameters.**

| Parameter | Trial 1 | Trial 2 | Trial 3 | Trial 4 | Trial 5 | Trial 6 |
|---|---|---|---|---|---|---|
| Extruded | yes | yes | no | no | no | yes |
| Sacc Time (h) | 3 | 3.5 | 4 | 7 | 5 | 5 |
| Incub pH | 4.2 | 5.1 | 4.3 | 4.6 | 3.05 | 3.15 |
| Incub Temp (C) | 29-30 | 29-30 | 29-30 | 26-27 | 29-30 | 29-30 |
| Aeration (L/L/min) | 0.25 | 0.25 | 0.25 | 0.5 | 0.5 | 0.5 |
| Incub Time (days) | 16 | 15 | 3.5 | 4.5 | 2 | 2.5 |
| Solids Recovery (%) | 20 | 16 | 48 | 48 | 60 | 64 |
| Solids % Protein | 75.18 | 75.04 | 63.93 | 61.5 | 61.61 | 56.86 |
| Trypsin Inhibitors (TIU) | 0 | 0 | NA | NA | 16,750 | 6,538 |
| Supernatant % Solids | 2.5 | 5 | 2.1 | 3.89 | 2.39 | 3.4 |
| Supernatant % Protein | 78.16 | 80 | 71.66 | 61.47 | NA | NA |

From the HQSPC yields and protein levels, the following were noted: 1) an incubation pH of 3-3.5 and temperature of 30-32° C, along with high aeration maximized growth of *A. pullulans* and minimized pullulan production, 2) an incubation time of 4-5 days was optimal for protein content and solids recovery, 3) longer incubation times increased protein content, but substantially reduced solids recovery, 4) shorter incubation times maintained high solids recovery, but limited protein content, and 5) due to the lack of stachyose and raffinose in the end product, extrusion and/or reduced (omitted) enzymatic saccharification may be possible.

Preliminary bench-scale trials in the 5L bioreactors were conducted to optimize process conditions. A 10% solid loading rate of extruded white flake was used and saccharified for 24h, followed by inoculation with *A. pullulans* and incubated at pH 5, 0.5 L/L/min aeration, 200 rpm agitation for 10 days. The extended incubation time was tested to establish optimal harvest window to maximize both percent solids recovery and protein content in the solids. Samples (100 ml) were removed daily and, on alternate days, were subjected to the following:

Precipitating all solids with ethanol, centrifuging and drying solids, measuring residual solids in the resulting supernatant.

Centrifuging the broth first to recover solids, drying the solids, precipitating the pullulan from the resulting supernatant and drying.

The ethanol precipitation first method recovered about 97% of the solids (soybean solids, cells and gum) using a lab centrifuge (10,000g), with about 3% solids remaining in the fluid phase. The centrifugation first method recovered about 81.7% of solids (soybean solids and cells), with ethanol precipitation of the supernatant recovering about 14.8% solids (exopolysaccharide), and about 3.5% solids remaining in the fluid.

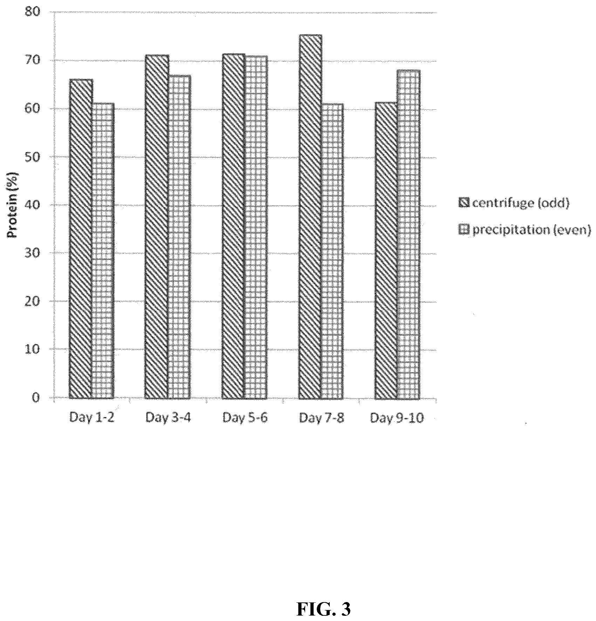

Through these bench scale trials, levels of protein, pullulan, and total solids that could be recovered each day were measured. It was expected that as incubation proceeded, protein and pullulan levels would increase, but that total solids recovered would decrease as some nutrients were catabolized into water and $CO_2$. Average protein levels of the solids from three replications are shown in FIG. 3. Protein levels reached 70% by day 3-5, while total solids recovered begin dropping by day 5-6. Thus it appears that a 4-5 day incubation time may be optimal.

**Example 2. Product comparison between precipitation (ppt) method, 3x wash and 1x wash methods.**

HQPC from soy bean was obtained by the methods as substantially recited above and as illustrated in FIGs 6-9, for both a 3x wash and 1x wash cycle. Comparison of resulting compositions for the ppt method (HQSPC Trial 5 and Trail 6), 3x wash and 1x (HQPC) wash are shown in Table 2.

**Table 2. Composition of the resulting proteins concentrates from ppt method, 3x and 1x wash methods (g/100 g, dry matter basis (dmb)).**

| Protein Source | HQSPC Trial 5 | HQSPC Trial 6 | HQPC 3x Wash | HQPC 1x Wash |
|---|---|---|---|---|
| **Proximate Components** | | | | |
| Protein | 61.61 | 56.86 | 75.30 | 69.17 |
| Moisture* | 5.14 | 7.89 | 3.87 | 4.42 |
| Lipid | 1.70 | 1.26 | 2.73 | 1.89 |
| Crude Fiber | 0.81 | 4.86 | 4.84 | 7.23 |
| Ash | 8.82 | 5.21 | 1.81 | 2.37 |
| **Amino Acids** | | | | |
| Alanine | 2.71 | 2.66 | 2.83 | 2.92 |
| Arginine | 2.44 | 3.65 | 4.53 | 4.23 |
| Aspartic Acid | 6.72 | 6.45 | 7.77 | 7.15 |
| Cystine | 0.87 | 0.88 | 0.91 | 1.00 |
| Glutamic Acid | 8.70 | 8.85 | 11.82 | 10.76 |
| Glycine | 2.67 | 2.51 | 2.70 | 2.67 |
| Histidine | 1.41 | 1.40 | 1.66 | 1.62 |
| Hydroxylysine | 0.81 | 0.10 | ND | ND |
| Hydroxyproline | 0.10 | 0.07 | ND | ND |
| Isoleucine | 2.89 | 2.92 | 3.23 | 2.79 |
| Lanthionine | 0.00 | 0.00 | ND | ND |
| Leucine | 4.64 | 4.87 | 5.37 | 5.87 |
| Lysine | 3.47 | 3.41 | 3.77 | 3.98 |
| Methionine | 0.83 | 0.90 | 0.84 | 0.95 |
| Ornithine | 0.14 | 0.04 | ND | ND |
| Phenylalanine | 2.89 | 3.08 | 3.60 | 3.89 |
| Proline | 3.17 | 2.92 | 3.46 | 5.80 |
| Serine | 2.28 | 2.73 | 3.41 | 3.79 |
| Taurine | 0.09 | 0.10 | ND | ND |
| Threonine | 2.36 | 2.31 | 2.63 | 2.62 |
| Tryptophan | 0.79 | 0.82 | 0.79 | 0.90 |
| Tyrosine | 1.98 | 2.25 | 2.59 | 2.74 |
| Valine | 3.13 | 3.10 | 3.33 | 3.17 |

(continued)

| Oligosaccharides | | | | |
|---|---|---|---|---|
| Raffinose | -- | 0.00 | 0.07 | 0.12 |
| Stachyose | -- | 0.24 | 0.11 | 0.18 |
| | | | | |
| **Phytic Acid** | -- | 0.23 | 0.12 | 0.08 |

**Example 3. Hydrolysis of soybean meal by temperature.**

A 10% (w/v) soybean meal slurry was prepared using hexane extracted soybean meal in water. The pH of the slurry was set at 4.5 and the slurry was agitated to obtain mixing. The soybean slurry was heated at a temperature of 100°C before fermentation. The heated mash was incubated with the fermentative organism. The heated mash with no fermentative organism was treated with FLAVORZYME® (Protease from *Aspergillus niger*, purchased from Sigma) at a loading rate of 30mg/g soybean meal and was used as control. The sample was incubated at 30°C for 12 hours. Post incubation, the samples were heated at 80°C for 2 minutes to inactivate the protease.

The mash was heated to 100°C for 1.5 minutes. The samples were centrifuged at 4000 rpm for 10 seconds. N-acetyl cysteine (3.33% w/v) was prepared in boric acid buffer (0.12 M and pH 10.4). 16.67$\mu$L of the sample supernatant was added to 1000mL of N-acetyl cysteine. The absorbance was measured at 340nm. 6% (w/v) OPA solution was prepared in 96% (v/v) ethanol solution. 10 $\mu$L of the OPA solution was added to 305 $\mu$L of sample. The sample with OPA solution was incubated at room temperature for 15 minutes. The absorbance was measured at 340 nm. The following calculation was used to calculate the relative degree of hydrolysis (%) [A340$_{test}$ (15)-A340$_{test}$ (0)] $\times$100/ [A340$_{control}$ (15)-A340$_{contol}$ (0)]. See Table 3.

**Table 3. Relative Degree of hydrolysis of soybean meal slurry by temperature.**

| Sample | Description | % DH |
|---|---|---|
| Control | FLAVORZYME® treated | 100 % |
| 0 Hour | No heat treatment | 2 |
| 0.025 Hour | Heat treated (100°C | 18.1 |
| | for 1.5 minutes) | |

**Example 4. Hydrolysis of the Soybean meal slurry by fermentation.**

A 10% (w/v) soybean meal slurry was prepared using hexane extracted soybean meal in water. The pH of the slurry was set at 4.5 and the slurry was agitated to obtain mixing. The soybean slurry was heated at a temperature of 100°C before fermentation. The heated mash was incubated with the fermentative organism. The heated mash with no fermentative organism was treated with FLAVORZYME® at a loading rate of 30mg/g soybean meal and was used as control. The sample was incubated at 30°C for 12 hours. Post incubation, the samples were heated at 80°C for 2 minutes to inactivate the protease.

Fermentation samples were collected at 0, 2, 4, 6, 8, 10 and 12 hours. The samples were centrifuged at 4000 rpm for 10 seconds. N-acetyl cysteine (3.33% w/v) was prepared in boric acid buffer (0.12 M and pH 10.4). 16.67$\mu$L of the sample supernatant was added to 1000mL of N-acetyl cysteine. The absorbance was measured at 340nm. 6% (w/v) OPA solution was prepared in 96% (v/v) ethanol solution. 10 $\mu$L of the OPA solution was added to 305 $\mu$L of sample. The sample with OPA solution was incubated at room temperature for 15 minutes. The absorbance was measured at 340 nm. The following calculation was used to calculated relative degree of hydrolysis (%) [A340$_{test}$ (15)-A340$_{test}$ (0)] $\times$100/ [A340$_{control}$ (15)-A340$_{contol}$ (0)]. See Table 4.

**Table 4. Relative Degree of hydrolysis of soybean meal slurry by fermentation.**

| Sample | Description | % DH |
|---|---|---|
| Control | FLAVORZYME® treated | 100 % |
| 0 Hour | Fermented | 5 |
| 2 Hour | Fermented | 12 |

(continued)

| Sample | Description | % DH |
|--------|-------------|------|
| 4 Hour | Fermented | 13 |
| 6 Hour | Fermented | 16 |
| 8 Hour | Fermented | 19 |
| 10 Hour | Fermented | 26.1 |
| 12 Hour | Fermented | 38 |

**Example 5. Hydrolysis of the Soybean meal slurry prepared in centrate by fermentation.**

A 10% (w/v) soybean meal slurry was prepared using hexane extracted soybean meal in centrate from solid-liquid separation. The pH of the slurry was set at 4.5 and the slurry was agitated to obtain mixing. The soybean slurry was heated at a temperature of 100°C before fermentation. The heated mash was incubated with the fermentative organism. The heated mash with no fermentative organism was treated with FLAVORZYME® at a loading rate of 30mg/g soybean meal was used as control. The sample was incubated at 30°C for 12 hours. Post incubation the samples were heated at 80°C for 2 minutes to inactive the protease.

Fermentation samples were collected at 0, 2, 4, 6, 8, 10 and 12 hours. The samples were centrifuged at 4000 rpm for 10 seconds. N-acetyl cysteine (3.34% w/v) was prepared in boric acid buffer (0.12 M and pH 10.4). 16.67$\mu$L of the sample supernatant was added to 1000mL of N-acetyl cysteine. The absorbance was measured at 340nm. 6% (w/v) OPA solution was prepared in 96% (v/v) ethanol solution. 10 $\mu$L of the OPA solution was added to 305 $\mu$L of solution A. The sample with OPA solution was incubated at room temperature for 15 minutes. The absorbance was measured at 340 nm. The following calculation was used to calculated relative degree of hydrolysis (%) [A340$_{test}$ (15)-A340$_{test}$ (0)] $\times$100/ [A340$_{control}$ (15)-A340$_{contol}$ (0)]. See Table 5.

**Table 5. Relative Degree of hydrolysis of soybean meal slurry in centrate by fermentation.**

| Sample | Description | % DH |
|--------|-------------|------|
| Control | FLAVORZYME® treated | 100 |
| 0 Hour | Fermented | 14.2 |
| 2 Hour | Fermented | 20.0 |
| 4 Hour | Fermented | 22.4 |
| 6 Hour | Fermented | 23.0 |
| 8 Hour | Fermented | 22.8 |
| 10 Hour | Fermented | 25.4 |
| 12 Hour | Fermented | 28.6 |

Example 5: Hydrolysis of the Soybean meal slurry with phytase addition by fermentation

A 10% (w/v) soybean meal slurry was prepared using hexane extracted soybean meal in water. The pH of the slurry was set at 4.5 and the slurry was agitated to obtain mixing. The soybean slurry was heated at a temperature of 100°C before fermentation. The heated mash was incubated with the fermentative organism. The heated mash with no fermentative organism was treated with FLAVORZYME® at a loading rate of 30mg/g soybean meal was used as control. The sample was incubated at 30°C for 12 hours. Post incubation the samples were heated at 80°C for 2 minutes to inactivate the protease.

Fermentation samples were collected at 0, 2, 4, 6, 8, 10 and 12 hours. 8, 10 and 12 hour samples were also treated with phytase. The samples were centrifuged at 4000 rpm for 10 seconds. N-acetyl cysteine (3.34% w/v) was prepared in boric acid buffer (0.12 M and pH 10.4). 16.67$\mu$L of the sample supernatant was added to 1000mL of N-acetyl cysteine. The absorbance was measured at 340nm. 6% (w/v) OPA solution was prepared in 96% (v/v) ethanol solution. 10 $\mu$L of the OPA solution was added to 305 $\mu$L of solution A. The sample with OPA solution was incubated at room temperature for 15 minutes. The absorbance was measured at 340 nm. The following calculation was used to calculated relative degree of hydrolysis (%) [A340$_{test}$ (15)-A340$_{test}$ (0)] $\times$100/ [A340$_{control}$ (15)-A340$_{contol}$ (0)]. See Table 6.

**Table 6. reflects the relative Degree of hydrolysis of soybean meal slurry with phytase by fermentation.**

| Sample | Description | % DH |
|---|---|---|
| Control | FLAVORZYME® treated | 100 |
| 0 Hour | Fermented | 5 |
| 2 Hour | Fermented | 12 |
| 4 Hour | Fermented | 13 |
| 6 Hour | Fermented | 16 |
| 8 Hour | Fermented | 19 |
| 8' Hour | Fermented and Phytase treated | 40 |
| 10 Hour | Fermented | 28 |
| 10' Hour | Fermented and Phytase treated | 60 |
| 12 Hour | Fermented | 38 |
| 12' Hour | Fermented and Phytase treated | 56 |

**Example 6. Rainbow trout, Barramundi and Coho Salmon Feeding Trials.**

Long and short-term feeding trials were completed using multiple lots of Rainbow trout (*Oncorhynchus mykiss*), Barramundi (*Lates calcarifer*) and Coho Salmon (*Oncorhynchus kisutch*). Fish in all trials were fed either a commercial control feed, or feeds utilizing high inclusion (25-35%) of HQPC made as described in Example 2 (3x wash). All feeds were formulated using commercial feed formulation software and manufactured using commercial extrusion methods. All chemical analysis (proximate analysis and mineral composition) of feeds were analyzed using 3rd party laboratories (Midwest Laboratories, Omaha, NE).

Grow out trials (~average individual initial weight 185 g-1000g harvest weight) were conducted using a RAS composed of 3.41 m$^3$ (900 gal) tanks. The RAS consisted of Cornell-style dual drain tanks, solids settling tanks, mechanical drum filtration, moving bed bioreactor (MBBR), UV sterilization, chilling, and oxygen injection. Water quality was monitored on a daily basis to ensure all parameters were within acceptable ranges.

At the day-0 starting sample, all fish were group-weighed by tank to determine biomass, and a sample of all the fish in 1 tank per treatment were anesthetized using 80 mg/L MS-222 and measured for fork length and weight. Group weights and identical individual fish sampling was completed at 3-week intervals throughout the 27-week trial. At week 16, 1 fish per tank (six fish per treatment) were sampled for general health evaluation including spleen, liver, visceral fat, and hematocrit.

Shrimp Feeding Trials

Larvae Development Trials

Approximately ninety million nauplii (Stage 5) of white leg shrimp *Litopenaeus vannamei* were produced at Sumacua's hatchery Choluteca, Honduras, and transferred to tanks containing 20 metric tons of salt water (28 ppt) in which all the experimental trials were conducted. Shrimp larvae were fed one of four diets containing HQPC (inclusion levels up to 70%) from Zoea 3 to Postlarva1 13 (PL13). Survival (%) was determined from extrapolation of aliquots in which all surviving larvae were counted at the end of the trial.

Growth out Trials

Pacific white leg shrimp *(L. vannamei)* (mean, 1.6 g) were randomly stocked (n=700) at a density of 20 animals per tank. Each treatment was randomly assigned to 5 replicate tanks. The experimental diets were offered 3 times per day (0800, 1200, and 1600 hr) for 42 days. All shrimp were fed the same ration of a control diet prior to the start of the trial. Upon the start of the trial (Day 1) shrimp were offered experimental diets and fed to apparent satiation. Total feed consumption was used to estimate feed conversion ratio. Tank biomass was recorded when stocking of shrimp occurred (Day 0) and again at 3-week intervals until completion of the trial. Total tank biomass measurements were used to calculate relative growth (RG), specific growth rate (SGR), and biomass gain over the course of the trial.

The experiment was conducted in an 8,246 L recirculating aquaculture system (RAS). The system was equipped with 35, 190 L semi-square tanks each equipped with a recirculating drain which withdrew water from the subsurface and a sludge drain which was affixed to the lowest point in the bottom at the center of the tank. Each tank had forced air diffusers fed by a blower, a water inlet flow bar that controlled the direction of current, and covers which provided darkness to half of the tank and netting which allowed light to penetrate the other half of the tank. The RAS was also equipped with a centrifugal water pump, bead filter, UV filter, biofilter, 3 solids settling sumps, clarifying sump, water inlet float valve, and a heater/chiller unit. The RAS replacement water was sourced with well water. Water flow to each tank was maintained at 6 to 7 L min$^{-1}$ and water temperature was maintained between 28 and 30°C. Dissolved oxygen was maintained above 5.0 mg L$^{-1}$, pH was held between 7 and 8, and salinity was maintained at 22 ppt throughout the study. Temperature, dissolved oxygen, and pH were monitored daily (0800 hr) while ammonia ($NH_3$) and nitrite ($NO_2$) were monitored weekly.

Challenge Trial

A trial was conducted to determine the effectiveness of diets containing HQPC in mitigating the severity and impact of the Early Mortality Syndrome/Acute Hepatopancreatic Necrosis Disease (EMS/AHPND) in shrimp. The trial was conducted at the Shrimp Vet Laboratory in Ho Chi Minh City, Vietnam and lasted 33 days including an adaption period for one day, twenty-one days of feeding period, one day of challenge, and following ten days of post-challenge.

Trials were carried out in 120L plastic tanks. All tanks were outfitted with an activated coral biological filter, aeration and covered with plastic cap to reduce the risk of cross contamination. Brackish water of 20 parts per thousand (ppt) of salinity was utilized in each trial. Shrimp were fed ad libitum with their respective diets with four meals per day during the trial. Feed consumption was recorded during the trial. Test diets included one basal diet and six treatment diets that contained HQPC (inclusion levels 10-30%). Feeding amount was adjusted depending on the biomass and actual feed consumption. Water quality parameters such as dissolved oxygen (DO), pH, and temperature were measured daily. Total ammonia nitrogen, nitrite, and alkalinity were measured twice a week.

Specific pathogen free (SPF) shrimp (*Litopenaeus vannamei*) were utilized in this trial, with the original genetics obtained from Hawaii broodstock that were checked for important pathogenic agents including *Enterocytozoon hepatopenaeid* (EHP), Whitespot syndrome virus (WSSV), *Taura syndrome virus* (TSV), *Infectious myonecrosis virus* (IMNV), and EMS/AHPND disease using PCR technique. Nauplii were reared in a strict biosecurity facility. Post-larvae were also checked again for important pathogenic agents including EHP, WSSV, TSV, IMNV, and EMS/AHPND disease using PCR technique. Post-larvae were grown in biosecurity conditions. One day prior to start the study, shrimp were weighed in-group to determine initial weight. The initial average shrimp weight was 0.56 + 0.04 grams.

An immersion challenge method was also used in this trial. Treatment tanks and positive controls, with total of 28 tanks were subjected to an immersion challenge. Tryptic Soy Broth +2% sodium chloride (TSB+) inoculated with a consistently virulent strain of *Vibrio parahaemolyticus,* incubated for 24 hours. The bacterial suspension was added into tanks to achieve the bacterial density measured by optical density absorbance (OD600 nm) at the density is expected to kill 90% in the positive control "LD90" within 10 days. Negative control (4 tanks total) was treated with sterile TSB+ added directly to the tanks. The challenge dosage was 3.25x105 CFU/mL which was lethal dose 90% (LD90). Standard histopathology, H&F stain was conducted in tissues of shrimp.

Fish Feeding Trials

ANCOVA results for multiple trials indicate that the control fish were significantly smaller and affected by both treatment (P=0.002) and week (P<0.001). In addition, there was no significant difference in FCR between the treatments or weeks (Figure 12). An analysis of covariance (treatment x week) revealed the vast majority of differences coming from the week rather than treatment feed. However, there were significant differences in average weight per fish for both treatment (P=0.046) and week (P<0.001) indicating fish fed the HQPC based feed were consistently larger than the fish fed the control feed. There were also significant differences in FCR for both treatment (P=0.001) and week (P<0.001) indicating fish fed the HQPC based feed consistently utilized feed more efficiently than the fish fed the control feed. The overall results of this study are similar to other experiments feeding fermented soybean meals to rainbow trout. Similarly, enteritis was not observed in these studies and no negative effects were found in the distal intestine of the rainbow trout fed HQPC based feeds.

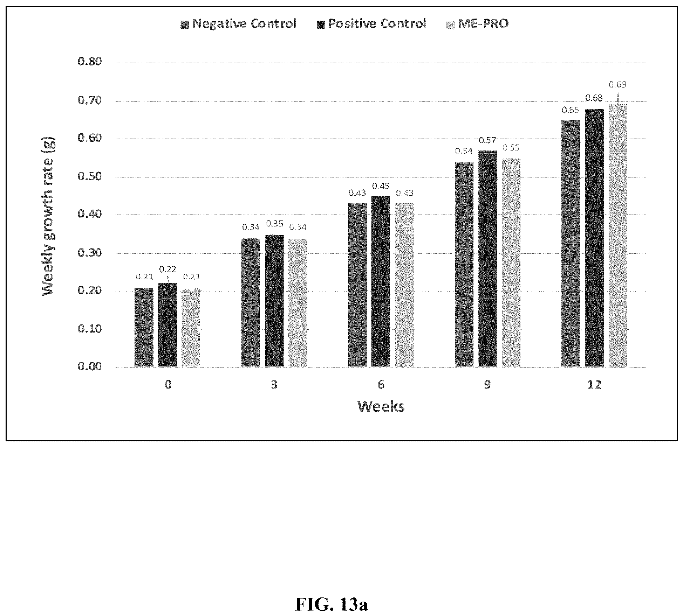

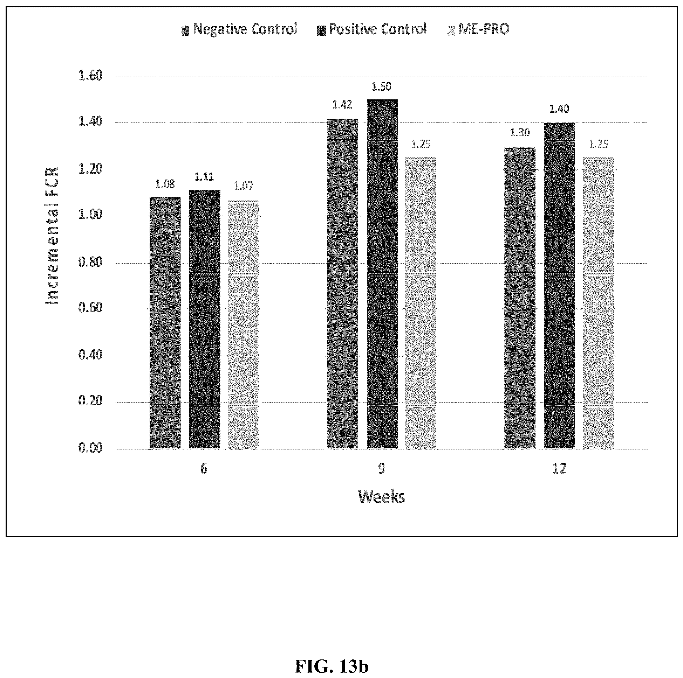

In a second and similar trial, fish fed the HQPC based feeds had significantly and consistently lower FCR (~1.0) than the control feed (~1.25) (Figures 13a, 13b). ANCOVA revealed a significant influence of the treatment (P<0.001) as well as week (P<0.001). Differences in average weight per fish were attributed to week (P<0.001) and not different between treatments (P=0.305). There were no significant differences in K-value (P=0.758), splenosomatic index (P=0.998), hepatosomatic index (P=0.475) or viscerosomatic index (P=0.411). Fish fed HQPC based feeds had significantly larger viscerosomatic index (P=0.040) and lower Hematocrit (P=0.005).

Shrimp Feeding Trials

Larvae Development Trials

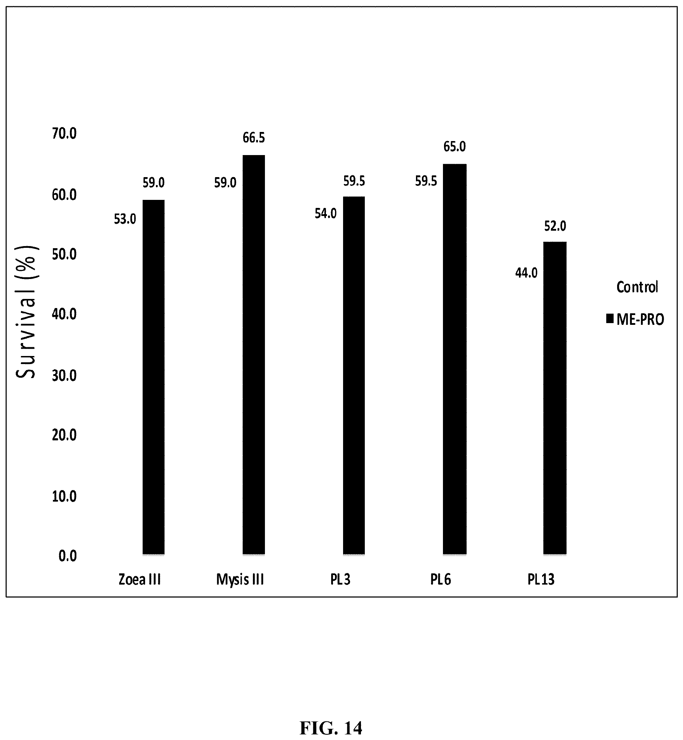

The inclusion of different percentages of HQPC in hatchery diets showed promising results, improving several productivity parameters, including survival of the larval stages (Figure 14).

Growth out Trials

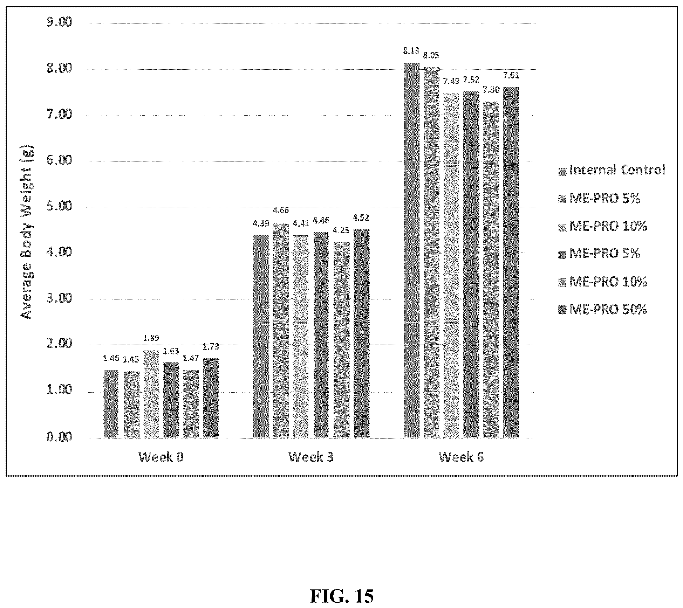

Weekly growth rates of shrimp fed on HQPC at levels up to 50% were not significantly different from those fed commercial feeds (Figure 15). While shrimp growth can be affected by numerous other factors such as water quality conditions and genetics, based on the observed response of the shrimp from the trial, there were no differences across the HQPC treatments, confirming the use of HQPC in commercial feed formulations. The results of the growth trial revealed that the higher apparent digestibility coefficient of HQPC along with higher amino acid digestibility resulted in better growth of the animals.

Challenge Trial

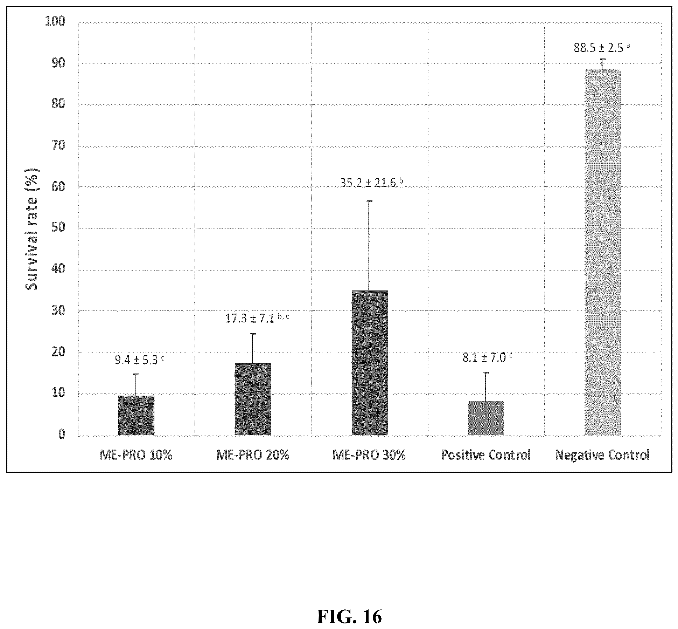

The results obtained indicated that the application of different inclusion levels has positive effects on the improvement of survival rate of the EMS-infected shrimp. Based on the results, the improvement of survival was observed in all treatments with diets containing HQPC. The trial indicated that the application of 30% of HQPC has positive effects on the improvement of survival rate of the EMS-infected shrimp (Figure 16).

Water Quality

Formulated feeds are the major driver allowing intensification of production but are also the major source of N and P in RAS. In addition, phosphorous is one of the most critical minerals when formulating aquaculture feeds for RAS projects. On average, HQPC contains 0.4% phosphorous (Table 7) and phytic acid concentration on the final product is on average less than 0.12 (g/100g).

**Table 7. Comparison of phosphorus content (% dry matter), phosphorus availability (%), and phosphorus discharged (% dry matter).**

| Protein Source | Phosphorous (% dmb) | Phosphorous Availability (%) | Phosphorous Discharged (% dmb) |
|---|---|---|---|
| HQPC | 0.4 | 90 | 0.05 |
| Fishmeal | 4.0 | 50 | 1.50 |
| Poultry meal | 2.3 | 50 | 1.00 |
| Soybean meal | 0.7 | 12 | 0.6 |

After 18 months of continuously using feeds containing HQPC data taken at a commercial trout operation showed a reduction in phosphorus discharge of 69% with initial phosphorus discharge levels of 0.21 mg/liter to levels of 0.065 mg/liter. During the shrimp growth and challenge trials, water quality parameters were recorded daily. Values of water quality parameters (temperature, DO, pH, TAN, nitrite, and alkalinity) are presented (Table 8).

**Table 8. Water quality parameters. Values (expressed as mean $\pm$ SE) within the same row sharing a common superscript are not significantly different (P>0.05).**

| Treatment | HQPC 10% | HWPC 20% | HQPC 30% | Positive Control | Negative Control |
|---|---|---|---|---|---|
| Temp (°C) | 27.53 $\pm$ 0.54[a] | 27.38 $\pm$ 0.49[a] | 27.49 $\pm$ 0.50[a] | 27.39 $\pm$ 0.51[a] | 27.45 $\pm$ 0.46[a] |
| DO (mg/L) | 6.19 $\pm$ 0.06[a] | 6.19 $\pm$ 0.06[a] | 6.19 $\pm$ 0.06[a] | 6.20 $\pm$ 0.06[a] | 6.18 $\pm$ 0.05[a] |
| pH | 7.78 $\pm$ 0.05[a] | 7.79 $\pm$ 0.05[a] | 7.78 $\pm$ 0.05[a] | 7.79 $\pm$ 0.05[a] | 7.79 $\pm$ 0.06[a] |
| Alkalinity (ppm) | 124.44 $\pm$ 5.27[a] | 125.56 $\pm$ 5.27[a] | 124.44 $\pm$ 5.27[a] | 124.44 $\pm$ 7.26[a] | 124.33 $\pm$ 5.27[a] |
| TAN (ppm) | 0.28 $\pm$ 0.26[a] | 0.22 $\pm$ 0.26[a] | 0.112 $\pm$ 0.22[a] | 0.22 $\pm$ 0.26[a] | 0.22 $\pm$ 0.26[a] |
| Nitrite (ppm) | 3.50 $\pm$ 2.26[a] | 3.61 $\pm$ 2.15[a] | 3.61 $\pm$ 2.15[a] | 3.61 $\pm$ 2.15[a] | 3.61 $\pm$ 2.15[a] |
| Salinility | 20.00 $\pm$ 0.00[a] | 20.00 $\pm$ 0.00[a] | 20.00 $\pm$ 0.00[a] | 20.00 $\pm$ 0.00[a] | 20.00 $\pm$ 0.00[a] |

Comparing fish trials testing HQPC and fishmeal water quality measurements have shown particular differences. Total dissolved solids in RAS water for HQPC and fishmeal treatments were 774 and 822 ppm respectively. Turbidity (NTU) measured in system tanks showed that HQPC (0.4) was slightly better than the fishmeal control (0.6).

The digestibility study was conducted at the Aquaculture Laboratory (LAM), Oceanographic Institute, University of São Paulo (USP; São Paulo, Brazil). Samples of two soybean products were provided by Prairie AquaTech (South Dakota, USA). Samples of the soy products - non-GMO soybean meal, SBM (46.8 percent crude protein, CP) and HQPC (74.6 percent CP) - had appropriate particle size (> 150 microns) and were analyzed for determination of their degree of protein hydrolysis (DH, percent).

These samples were tested for in vitro protein digestion with standardized digestive enzymes recovered from the hepatopancreas of pond-farmed Pacific white shrimp (10 grams average weight). The hydrolysis with shrimp enzymes involved the suspension of 80 mg of ingredient protein in distilled water, with pH of the suspension set at 8.0 following the addition of the hepatopancreas enzyme extract for hydrolysis.

The pH shift and hydrolysis monitoring were automatically performed by commercial, software-controlled potentiometric titrators in temperature-controlled devices (30 $\pm$ 0.6 degrees-C). At this reaction pH (= 8.0), the enzymatic breakage of ingredient peptide bonds produces a slight reduction in reaction pH that is registered and automatically neutralized by the titrator with addition of sodium hydroxide, NaOH. At the end of the reaction the amount of titrant (NaOH) expended is proportional to the number of peptide bonds cleaved and a quantitative value provided.

No buffers or other chemicals were used in the analysis. If determined significant, then blank DH values were computed for the calculation of net DH of ingredient protein.

Results of the study show a significant difference in the degree of hydrolysis (DH) of the test ingredients with shrimp digestive enzymes (Table 9), with the DH value obtained for HQPC exhibiting 42 percent more hydrolysable protein than soybean meal, and 33 percent more than soy protein concentrate.

Typical DH values of SBM ranged between 4.0 percent and 4.2 percent for dehulled or non-dehulled samples, respectively. Results from comparable pH stat studies on a set of more than 40 samples of SBMs from the main producer countries (India, Argentina, United States and Brazil), have shown DH values between 3.74 and 4.43 percent.

Appropriate screening of novel ingredients is required to assess their potential nutritional value and variability. Various studies have shown that in vitro digestion of ingredients by enzymes from the target shrimp or fish species is associated with apparent protein digestibility (APD). For almost half a century, the pH-stat method has been used to monitor the effects of heat treatment on the initial rates of trypsin proteolysis of soy protein. Previous research has also demonstrated the relationship between apparent protein digestibility (APD) and in vitro DH in juvenile Pacific white shrimp.

The average in vivo apparent digestibility coefficient for crude protein in *L. vannamei* is 85 to 90 percent. In the trial conducted, the degree of hydrolysis (DH) of HQPC was estimated to be 7.18 percent and the predicted apparent protein digestibility (PPD) at 93.1percent. When compared with other studies, HQPC values for DH and PPD are the highest determined among over 150 ingredients tested - higher than various fish meals, soy protein concentrates and non-GM soybean meal (Table 9).

**Table 9. *In vitro* protein digestion of ingredient samples (DH, degree of protein hydrolysis) with Pacific white shrimp (*L. vannamei*) digestive enzyme extracts.**

| Ingredient | PPD** (%)** | DH (%)* |
|---|---|---|
| Fishmeal (anchovy) | 87.90 | 3.08 |
| Fishmeal (anchovy, Peru) | 88.50 | 3.13 |
| Fishmeal (herring) | 90.10 | 3.42 |
| Poultry byproduct meal | 78.70 | 5.01 |
| Distiller's dried grains with solubles | 78.50 | 3.10 |
| Corn gluten meal | 59.10 | 2.11 |
| Soybean meal (solvent extract) | 92.90 | 4.15 |
| Soybean meal (full fat, extruded) | 87.10 | 2.72 |
| Soy protein concentrate | 89.60 | 4.80 |
| Non-GM soybean meal | 88.50 | 4.54[a] |
| ME-PRO® | 93.10 | 7.18[b] |

* Mean values with different superscripts were found significantly different (P < 0.05).

** Predicted apparent protein digestibility (PPD) calculated by a regression between *in vivo* apparent protein digestibility and *in vitro* protein digestion with Pacific white shrimp digestive enzymes (DH) of different feed ingredients

In addition to multiple previous studies, this evaluation showed the potential of the commercial product HQPC as a replacement ingredient for fishmeal in aquaculture diets regarding its in vitro digestibility. Results demonstrate that this ingredient product has significantly higher levels of hydrolysable protein, and also a higher predicted apparent protein Digestibility than various soybean meal ingredients.

All of the references cited herein are incorporated by reference in their entireties.

From the above discussion, one skilled in the art can ascertain the essential characteristics of the invention, and without departing from the spirit and scope thereof, can make various changes and modifications of the embodiments to adapt to various uses and conditions. Thus, various modifications of the embodiments, in addition to those shown and described herein, will be apparent to those skilled in the art from the foregoing description. Such modifications are also intended to fall within the scope of the appended claims.

**Claims**

1.  A method of treating plant-based material comprising:

    a) transferring said plant-based material to a first mix-tank (102(a)), wherein said plant-based material is mixed with one or more first solvents to produce a washed mash;
    b) separating the washed mash into at least one centrate and a washed cake;
    c) transferring said washed cake to one or more second mix-tanks (102(b)-102(d)), wherein one or more second solvents are mixed with said washed cake to produce a washed cake suspension;
    d) transferring said washed cake suspension to one or more fermenters (104), wherein said transferred washed cake suspension is inoculated with at least one microbe, and wherein said inoculated washed cake suspension is incubated for a sufficient time to produce a fermented mixture;
    e) optionally heating said fermented mixture for a time sufficient to achieve a degree of hydrolysis (DH) of at least 10% of the proteins therein;
    f) separating said fermented mixture into a fermented centrate and a fermented cake by hydrodynamic force;
    g) transferring the fermented centrate to:

        (i) a first mix-tank (102(a)) and/or
        (ii) one or more second mix-tanks (102(b)-102(d)),
        wherein a mix-tank comprises said plant-based material or said washed cake, and wherein steps (c)-(f) and
        h) are repeated at least one (1) time for sub-steps (i) or
        (ii); and

    h) drying said fermented cake,

    wherein the resulting dried fermented cake has higher protein content compared to said transferred plant-based material.

2.  The method of claim 1, wherein the at least one microbe is selected from the group consisting of *Kluyveromyces, Pichia spp,* Lactic acid bacteria, *Trichoderma reesei, Pleurotus ostreatus,* and *Rhizopus spp.*

3.  The method of claim 1, further comprising transferring said at least one centrate of step (b) to one or more of said mix-tanks (102(a)-102(d)) prior to inoculation.

4.  The method of claim 3, further comprising recycling said centrates, wherein recycling of said centrates: a) reduces the amount of fresh solvent added to a first mix-tank (102(a) and/or one or more second mix-tanks (102(b)-102(d)) and/or b) increases yield and recovery of protein.

5.  The method of claim 1, wherein said method does not include addition of cellulose deconstructing enzymes.

6.  The method of claim 1, further comprising heating said washed cake suspension prior to transfer to one or more fermenters (104).

7.  The method of claim 6, wherein the washed cake suspension is heated to greater than 100°C.

8.  The method of claim 3, wherein the fermentation centrate is transferred to said first mix tank (102(a)).

9.  The method of claim 3, wherein the centrates and cakes are produced by hydrodynamic force, wherein the hydrodynamic force is filtration, ultrafiltration, or nanofiltration, and wherein the method comprises a system of four (4) mix tanks (102(a)-102(d)) and four (4) centrifuges (103(a)-103(d)) in series, wherein the fermentation centrate of mix-tank 4 (102(d)) is transferred to mix-tank 3 (102(c)), the centrate of mix-tank 3 (102(c)) is transferred to mix-tank 2 (102(b)), and the centrate of mix-tank 2 (102(b)) is transferred to mix-tank 1 (102(a) prior to a second fermentation.

10. The method of claim 1, wherein the solvent is selected from one or more of water, an acid, an aqueous enzyme mixture, antifomates or a combination thereof, wherein the aqueous enzyme mixture comprises phytase.

11. The method of claim 1, wherein the centrate from the first mix tank (102(a)), the fermented centrate, or both are transferred to at least one evaporator (107) producing a liquid protein condensate.

12. The method of claim 11, wherein said centrate is evaporated at a temperature of between about 60°C to 90°C and/or about 1 psia to 6 psia.

13. The method of claim 1, wherein the non-animal based protein concentrate is isolated from plant material from the group consisting of soybeans, sorghum, peanuts, pulses, Rapeseeds, oats, barley, rye, lupins, fava beans, canola, peas, sesame seeds, cottonseeds, palm kernels, barley, grape seeds, olives, safflowers, sunflowers, copra, corn, coconuts, linseed, hazelnuts, wheat, rice, potatoes, cassavas, legumes, camelina seeds, mustard seeds, germ meal, corn gluten meal, distillery/brewery by-products, and combinations thereof.

14. The method of claim 1, wherein drying is carried out at greater than 100°C.

15. The method of claim 1, further comprising adding one or more cellulose-deconstructing enzymes.

16. A concentrate produced by the method of claim 1, wherein the concentrate is selected from a solid protein concentrate or liquid protein concentrate.

17. The concentrate of claim 16, wherein said protein concentrate exhibits a degree of hydrolysis (DH) greater than 10%.

18. The concentrate of claim 16, wherein the plant-based material is selected from the group consisting of soybeans, sorghum, peanuts, pulses, Rapeseeds, oats, barley, rye, lupins, fava beans, canola, peas, sesame seeds, cottonseeds, palm kernels, barley, grape seeds, olives, safflowers, sunflowers, copra, corn, coconuts, linseed, hazelnuts, wheat, rice, potatoes, cassavas, legumes, camelina seeds, mustard seeds, germ meal, corn gluten meal, distillery/brewery by-products, and combinations thereof.

19. The concentrate of claim 18, wherein the plant material is from soybeans in the form of soy flakes or soy meal.

20. A feed or foodstuff comprising the concentrate of claim 16.

21. The foodstuff of claim 20, wherein said concentrate is combined with one or more meat substitutes.

22. The foodstuff of claim 21, wherein the meat substitute is selected from the group consisting of thawed and sliced frozen tofu, oncom, tempeh, tofu, tofurkey, faux turkey, paneer, glamorgan, breadfruit, sapal, eggplant, jackfruit, falafel, ganmodoki, and combinations thereof.

23. The foodstuff of claim 21, wherein the concentrate improves one or more of the sensory characteristics selected from texture, aroma, mouthfeel, bite, crunch, flavor, appearance, or combinations thereof, of said one or more meat substitutes compared to the same meat substitutes lacking said concentrate.

24. The foodstuff of claim 21, wherein said foodstuff is for human consumption.

25. The feed of claim 20, wherein the feed is formulated for animals selected from the group consisting of fin fish, shellfish, crustaceans, domestic animals, farm animals, and combinations thereof.

**FIG. 1**

**FIG. 2**

**FIG. 3**

**FIG. 4**

**FIG. 5**

# Production Process

FIG. 6

FIG. 7

# Centrate 4

**100(b)**

**103(c)**

**103(d)**

Evap.

Sep 3

**102(d)**

Sep 4

**108**

Mix 4

Fermentation

**104**

**Centrate 4**

**FIG. 8**

**100(c)**

FIG. 9

**FIG. 10**

FIG. 11

**FIG. 12**

**FIG. 13a**

**FIG. 13b**

**FIG. 14**

**FIG. 15**

**FIG. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4800159 A

**Non-patent literature cited in the description**

- **CASWELL**. *J Agr Res Econ*, 1998, vol. 42, 409-474
- **LEATHERS et al.** *J Indus Micro*, 1988, vol. 3, 231-239
- **FAN et al.** *PLoS ONE*, 2016, vol. 11 (9), e0163145
- **WHITE et al.** *Aqua Mar Bio Eco*, 2020, 105